# EUROPEAN PATENT SPECIFICATION

(11) **EP 2 069 378 B1**
(45) Date of publication and mention of the grant of the patent: **05.10.2016**
(21) Application number: 07867206.0
(22) Date of filing: 05.10.2007
(51) Int. Cl.: C12N 9/64, C12N 9/50, A61K 39/00

(54) **DNA COMPOSITION FOR ELICITING AN IMMUNE RESPONSE AGAINST TUMOR-ASSOCIATED MACROPHAGES**
DNA-ZUSAMMENSETZUNG ZUR AUSLÖSUNG EINER IMMUNREAKTION GEGEN TUMOR-ASSOZIIERTE MAKROPHAGEN
COMPOSITION D'ADN POUR ÉLICITER UNE RÉPONSE IMMUNE CONTRE DES MACROPHAGES ASSOCIÉS AUX TUMEURS

(30) Priority: 06.10.2006 US 849927 P
(43) Date of publication of application: 17.06.2009
(73) Proprietor: THE SCRIPPS RESEARCH INSTITUTE, La Jolla, CA 92037 (US)
(72) Inventor: REISFELD, Ralph, A., La Jolla, CA 92037 (US); XIANG, Rong, San Diego, CA 92122 (US); LUO, Yunping, San Diego, CA 92122 (US)
(74) Representative: Schnappauf, Georg
(86) International application number: PCT/US2007/021414
(87) International publication number: WO 2008/054612

(56) References cited:
- WO-A-2007/064759
- LUO YUNPING ET AL: "Targeting tumor-associated macrophages as a novel strategy against breast cancer" JOURNAL OF CLINICAL INVESTIGATION, AMERICAN SOCIETY FOR CLINICAL INVESTIGATION, US, vol. 116, no. 8, 1 August 2006 (2006-08-01), pages 2132-2141, XP002536336 ISSN: 0021-9738
- ZHOU H, ET AL.: "T CELL-MEDIATED SUPPRESSION OF ANGIOGENESIS RESULTS IN TUMOR PROTECTIVE IMMUNITY" BLOOD, vol. 106, no. 6, 2005, pages 2026-2032, XP002548097
- XIANG R ET AL: "An autologous oral DNA vaccine protects against murine melanoma" PROCEEDINGS OF THE NATIONAL ACADEMY OF SCIENCES OF USA, NATIONAL ACADEMY OF SCIENCE, WASHINGTON, DC, US, vol. 97, no. 10, 9 May 2000 (2000-05-09), pages 5492-5497, XP002963882 ISSN: 0027-8424
- SUSANNA LEWÄ N ET AL: "A Legumain-based minigene vaccine targets the tumor stroma and suppresses breast cancer growth and angiogenesis" CANCER IMMUNOLOGY, IMMUNOTHERAPY, SPRINGER, BERLIN, DE, vol. 57, no. 4, 5 September 2007 (2007-09-05), pages 507-515, XP019586703 ISSN: 1432-0851
- WATTS ET AL.: 'Asparaginyl endopeptidase: case history of a class II MHC compartment protease' IMMUNOLOGICAL REVIEWS vol. 207, 2005, pages 218 - 228, XP008108558
- VOSKOGLOU-NOMIKOS ET AL.: 'Clinical predictive value of the in vitro cell line, human xenograft, and mouse allograft preclinical cancer models' CLINICAL CANCER RESEARCH vol. 9, 2003, pages 4227 - 4239, XP008108561

## Description

### GOVERNMENTAL RIGHTS

This invention was made with United States government support under Grant Nos. DAMD17-02-0137 and DAMD17-02-0562 from the Department of Defense, as well as Grant Nos. W81XWH-05-1-0091 and W81XWH-05-1-0318 from the Congressionally Directed Medical Research Program. The government has certain rights in this invention.

### FIELD OF THE INVENTION

This invention relates to deoxyribonucleic acid (DNA) compositions encoding a polypeptide comprising a plurality of fragments of legumain. This invention also relates to an article of manufacture comprising said DNA compositions.

### BACKGROUND OF THE INVENTION

Tumor-associated macrophages (TAMs) are associated with tumor progression and metastasis. A novel anti-tumor strategy is to immunize against molecules overexpressed by TAMs and thereby remodel the tumor microenvironment, which attracts these macrophages and mediates their function. (see Oosterling et al. 2005 J. Pathol. 207:147-155; Emens et al. 2005 Endocr. Relat Cancer 12:1-17). TAMs consist primarily of a polarized M2 (CD206⁺, F4/80⁺) macrophage population with little cytotoxicity for tumor cells because of their limited production of nitric oxide and proinflammatory cytokines (see Mills et al. 2000 J. Immunol. 164:6166-6173).

TAMs also possess poor antigen presenting capability and effectively suppress T cell activation. In fact, these macrophages of M2 phenotype actually promote tumor cell proliferation and metastasis by secreting a wide range of growth and pro-angiogenesis factors as well as metalloproteinases, and by their involvement in signaling circuits that regulate the function of fibroblasts in the tumor stroma (Mantovani et al. 2004, Novartis. Found. Symp. 256:137-145).

Currently, anti-TAM effects induced by small molecule inhibitors reportedly have contributed to tumor suppression (see Lewis et al. 2005 Am. J. Pathol. 167:627-635; and Mantovani et al. 2004, Eur. J. Cancer 40:1660-1667). For example, yondelis, an antineoplastic agent, has a selective cytotoxic effect on TAMs, thereby significantly reducing their production of IL6 and CCL2, which contribute to growth suppression of inflammation-associated human tumors (see Allavena et al. 2005, Cancer Res. 65:2964-2971). Another such example is provided by a biphosphonate compound, zoledronic acid, which suppresses MMP9 secretion by TAMs, thereby inhibiting tumor metalloproteinase activity and diminishing the association of VEGF with its tyrosine kinase receptors on proliferating endothelial cells (see Giraudo et al. 2004, J. Clin. Invest. 114:623-633).

In a different experimental model, the chemokine CCL5 was shown to be important in the recruitment of TAMs. An antagonist of this chemokine reduced the tumor infiltrate and slowed tumor growth (see Robinson et al. 2003, Cancer Res. 63:8360-8365). Hence, although the therapeutic targeting of TAMs is still a relatively new approach, initial clinical results are encouraging, as they suggest that targeting TAMs may complement more conventional cancer treatment regimens.

Legumain is an entirely novel evolutionary offshoot of the C13 family of cysteine proteases (see Ishii 1994, Methods Enzymol. 244:604-615), and is well conserved in plants and mammals, including humans. Legumain was first identified in plants as a processing enzyme of storage proteins during seed germination, and was subsequently identified in parasites and then in mammals. Legumain is a robust acidic cysteine endopeptidase with remarkably restricted specificity absolutely requiring an asparagine at the P1 site of its substrate sequence (see Chen et al. 1997, J. Biol. Chem. 272:8090-8098).

In the present application, the selection of legumain as a target for tumor therapy is based on the fact that the gene encoding this endopeptidase was found to be highly up-regulated in many murine and human tumor tissues, but absent or only present at very low levels in all normal tissues from which such tumors arise. Importantly, overexpression of legumain occurs under such stress conditions as tumor hypoxia, which leads to increased tumor progression, angiogenesis and metastasis. Luo et al. (Journal of Clinical Investigations 2006, 116: 2132-2141) describes a DNA vaccine encoding a full-length legumain and mentions that this vaccine is capable of inducing a CTL.

Legumain is a particularly preferred target endopeptidase for the compositions and methods described herein, due to the observation that legumain is heavily overexpressed by TAMs in murine breast tumor tissues, as confirmed by gene expression profiling and immunohistochemistry. TAMs have a particularly abundant expression of legumain in the tumor stroma. In contrast, classical macrophages of M1 phenotype, which perform important immune-surveillance and antigen presentation functions, do not express legumain. Consequently, targeting TAMs that overexpress legumain does not interfere with the biological functions of (M1) macrophages, including cytotoxicity and antigen presentation. The present invention provides DNA compositions to induce an immune response against TAMs that overexpress legumain, and which are useful for treatment of tumors and tumor metastases.

### SUMMARY OF THE INVENTION

A DNA composition of the present invention comprises a DNA minigene construct that encodes an immunogenic polypeptide comprising a plurality of legumain fragments, each of which is preceded by the three amino acids AAY, the polypeptide being expressed in tumor-associated cells, the immunogenic fragments being joined together serially by said three amino acids AAY between each successive fragment in the polypeptide, wherein the polypeptide is capable of eliciting an immune response against tumor-associated cells, and is expressible in immune cells, and wherein the DNA minigene construct is incorporated in a pharmaceutically acceptable carrier, wherein said immunogenic fragments have an amino acid sequence according to SEQ ID NOs: 16, 17 and 18. The DNA construct includes structural elements that facilitate the expression of the polypeptide in immune cells of a subject to which the DNA construct has been administered. The DNA construct is incorporated in a pharmaceutically acceptable carrier so that it can be administered to a patient. The composition can encode a single immunogenic fragment of the endopeptidase (e.g., an epitope of legumain), a polypeptide comprising two or more immunogenic fragments of the endopeptidase (i.e., an immunogenic polypeptide), an entire endopeptidase protein, or any portion thereof that will elicit an immune response in the subject. The DNA construct may encode for human legumain having the amino acid residue sequence consisting of SEQ ID NO: 2, a protein that has at least 80% sequence identity with SEQ ID NO: 2 (e.g., human legumain, porcine legumain, or mouse legumain), or an immunogenic fragment thereof, and which is expressible in immune cells of a subject to which the DNA construct is administered. The DNA constructs of the invention are useful for inhibiting tumor growth and tumor metastases.

The DNA construct can be a naked DNA construct, preferably in the form of a plasmid. Such naked DNA constructs can be incorporated in a liposome delivery vehicle, a polymeric delivery vehicle, or administered by electroporation, a gene gun, and the like, if desired. In some preferred embodiments the DNA construct is incorporated in an attenuated viral vector or an attenuated bacterial vector.

In a preferred embodiment, the DNA construct is incorporated in an attenuated bacterial vector, such as an attenuated *Salmonella typhimirium,* e.g., the doubly attenuated (*AroA , dam*⁻) strain of *Salmonella typhimurium.*

Optionally, the DNA composition can also comprise a DNA construct encoding an immune effector protein, such as a cytokine. Preferred cytokines include CCL21, IL-2, and CD40LT.

The immunogenic polypeptide encoded by the DNA minigene of the present invention is capable of eliciting an immune response against the tumor-associated cells, and is expressible in immune cells, and wherein the DNA minigene construct is incorporated in a pharmaceutically acceptable carrier. The immunogenic fragments are joined together serially by a linker peptide between each successive fragment in the polypeptide. The linker peptides described herein below typically are at least three amino acid residues in length and comprise the amino acid sequence AAA or AAY. As used herein below, the term "linker peptide" refers to a sequence of at least two amino acid residues, preferably at least three amino acid residues, which form an amino acid residue sequence that differs from the natural endopeptidase when linked together with the immunogenic fragments of the endopeptidase. Typically the combination of linker peptides and immunogenic fragments of the endopeptidase will comprise a polypeptide of less than about 100 amino acid residues in length, more preferably about 19 to about 62 amino acids in length (e.g., two to about five immunogenic fragments of 8 to 10 amino acids each, joined together by one to four linker peptides of three amino acids each). The DNA minigene constructs described herein can encode for immunogenic fragments of human legumain (SEQ ID NO: 2).

The DNA compositions of the present invention can act as vaccines that target tumor-associated macrophages that express a cysteine endopeptidase, such as legumain, providing a highly selective target for T cell-mediated cancer immunotherapy. The approach of targeting an endopeptidase such as legumain, which is expressed by tumor-associated macrophages has several advantages over therapies directed against antigens that are solely expressed by tumor cells themselves. For example, legumain is highly overexpressed in TAMs, and is thus not impaired by downregulated MHC-antigen expression, as is frequently the case in tumor cells. In addition, tumor cells often become increasingly resistant to T cell mediated killing due to defects in apoptosis signaling pathways, upregulation of antiapoptotic proteins, or immunosuppressive effects on cytotoxic T lymphocytes (CTLs). Targeting TAMs that express legumain allows for a therapeutic composition to treat a number of different malignancies, in contrast to therapies involving antigens that are expressed solely by specific tumor types.

The DNA compositions described herein can be used to break peripheral T cell tolerance against the legumain self-antigen by delivering its cDNA of DNA encoding one or more immunogenic fragments thereof, as an oral DNA composition with an attenuated bacterial delivery vector (e.g., an attenuated strain of *Salmonella typhimurium*). In such embodiments, the DNA composition is contacted with antigen presenting cells (APCs) in a secondary lymphoid organ, i.e., the Peyer's patches of the small intestine. In a prophylactic approach, the T cell-mediated antitumor immune response induced by vaccination with a DNA composition of the invention inhibited tumor growth in multiple murine tumor models. The present DNA compositions also significantly suppress the dissemination of established pulmonary metastases in a therapeutic model of CT26 colon carcinoma.

A preferred DNA composition comprises an attenuated *Salmonella* carrier, such as a doubly attenuated strain of *S. typhimurium,* e.g., the strain designated as RE 88, which includes the *dam*⁻ and *AroA*⁻ mutations, and is available from Remedyne Corporation (Goleta, CA). The attenuated *Salmonella* carrier may be transfected so that it includes the DNA construct encoding the endopeptidase (e.g., legumain) or a polypeptide encoding an immunogenic fragment thereof. The endopeptidase or polypeptide is expressible in immune cells of a mammal to which it is administered. The bacteria do not themselves express the legumain or polypeptide, but rather deliver the DNA to immune cells, such as macrophages and dendritic cells (DCs), which in turn express endopeptidase or the polypeptide comprising the immunogenic fragment thereof. Such compositions can provide prolonged antitumor effects in murine models. Furthermore, *in vivo* depletion experiments of T cells indicated the involvement of CD8⁺ but not CD4⁺ T cells in the immunogenic response associated with compositions encoding legumain and encoding polypeptides comprising immunogenic fragments of legumain. The observed cytotoxic effect mediated by CD8⁺ T cells *in vitro* was specifically directed against target TAMs that overexpress the legumain antigen.

The DNA compositions described herein also can incorporate DNA constructs that encode immune effector molecules as adjuvants for the composition. Such immune effector molecules include, for example, IL-2, an inducer of T cell proliferation, CCL21, a chemokine that chemo-attracts mature dendritic cells, and naive T cells, as well as CD40LT, a known inducer of dendritic cell maturation. The nucleic acids encoding the immune effector protein preferably are incorporated into a plasmid. The legumain and immune effector protein DNA constructs can be incorporated into the same plasmid or into two separate plasmids. The CTL-response induced against TAMs can inhibit the growth of a variety of tumors, and is not specific to a particular tumor type.

The present specification also discloses a method of inhibiting tumor growth and tumor metastases in a mammal comprising administering to a mammal a DNA composition of the invention in an amount sufficient to elicit an immune response against TAMs that express legumain.

The present specification discloses an effective combination therapeutic regimen that combines chemotherapy and treatment with a DNA composition of the invention. In this method embodiment various chemotherapeutic agents, such as doxorubicin, paclitaxel, and/or cyclophosphamide, which do not cause bone marrow suppression when administered at the maximum tolerable dose (MTD), are administered to the patient in conjunction with a DNA composition of the invention comprising a DNA construct that encodes a TAM-expressed endopeptidase, such as legumain or a polypeptide encoding an immunogenic portion thereof, preferably comprising a minigene construct that comprises at least two immunogenic fragments of the endopeptidase jointed together serially by a linker peptide between each successive immunogenic fragment in the polypeptide.

The present invention also relates to the use of the DNA compositions of the present invention in a method of inhibiting tumor growth or tumor metastases in a mammal (e.g., a human). Such uses can comprise the steps of administering to a mammal a DNA composition of the invention in an amount sufficient to elicit an immune response against TAMs in the mammal, which overexpress an endopeptidase such as legumain, and subsequently administering to the mammal an antitumor effective amount of an antitumor chemotherapeutic agent.

Preferably, the mammal treated is a human. In the method embodiments described herein, the DNA compositions can be administered enterally, such as by oral administration, or parenterally, such as by injection or intravenous infusion. Preferably, the compositions are administered orally. The compositions can be packaged in sealed containers and labeled with information useful for a clinician to effectively administer the composition.

The DNA compositions described herein are useful for treatment and prevention of a number of disease states. For example, a patient suffering from colorectal cancer, breast cancer, lung cancer, and the like, can benefit from immunization by the compositions of the present invention. The compositions of the present invention are also useful for investigating the role of legumain in various forms of cancer.

### BRIEF DESCRIPTION OF THE DRAWINGS

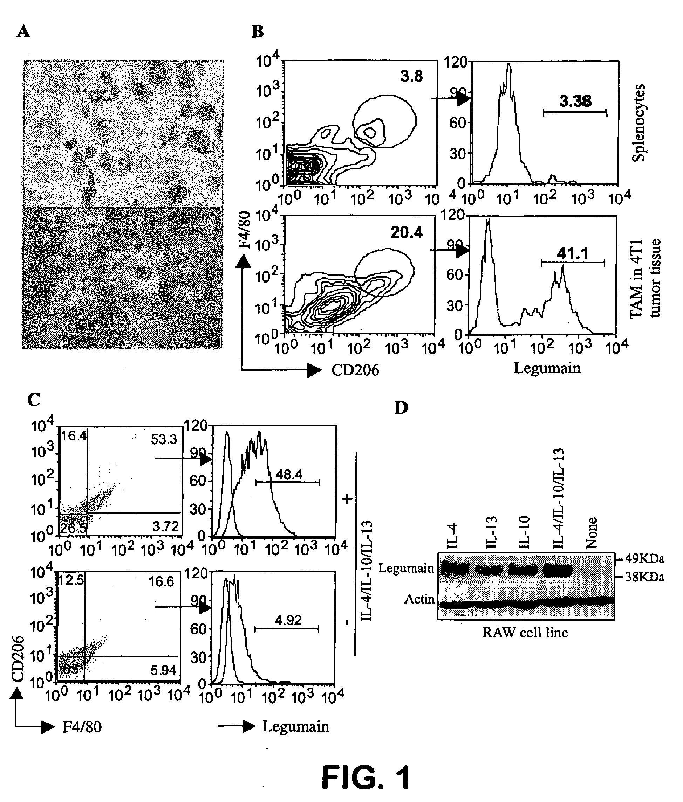

FIG. 1. Legumain is highly expressed on tumor-associated macrophages in the tumor stroma. (A) Legumain expression on TAMs was clearly evident as shown in Panel A. Tumor-infiltrating macrophages were visualized by H/E staining as indicated by arrows. Legumain expression is indicated by double staining with anti-legumain Ab combined with anti-CD68 Ab. (Magnification x35) (B) Increased legumain expression on TAMs was confirmed by flow cytometric analyses with double positive populations of CD206⁺/F4/80⁺ M2 macrophages that were isolated from fresh tumor tissue. (C) Multiple color flow cytometry demonstrated up-regulation of the M2 macrophage marker CD206 on RAW cells after being cultured with IL-4, IL-10 and IL-13 (10 ng/ml). Legumain was shown to be highly expressed on F4/80⁺/CD206⁺ positive RAW cells cultured with IL-4, IL-10 and IL-13 as indicated in the upper photo when compared with wild type RAW cells depicted in the lower photo. (D) Confirmation of legumain expression on RAW cells by Western blotting following stimulation with IL-4, IL-13 and I-10, either singularly or combined.

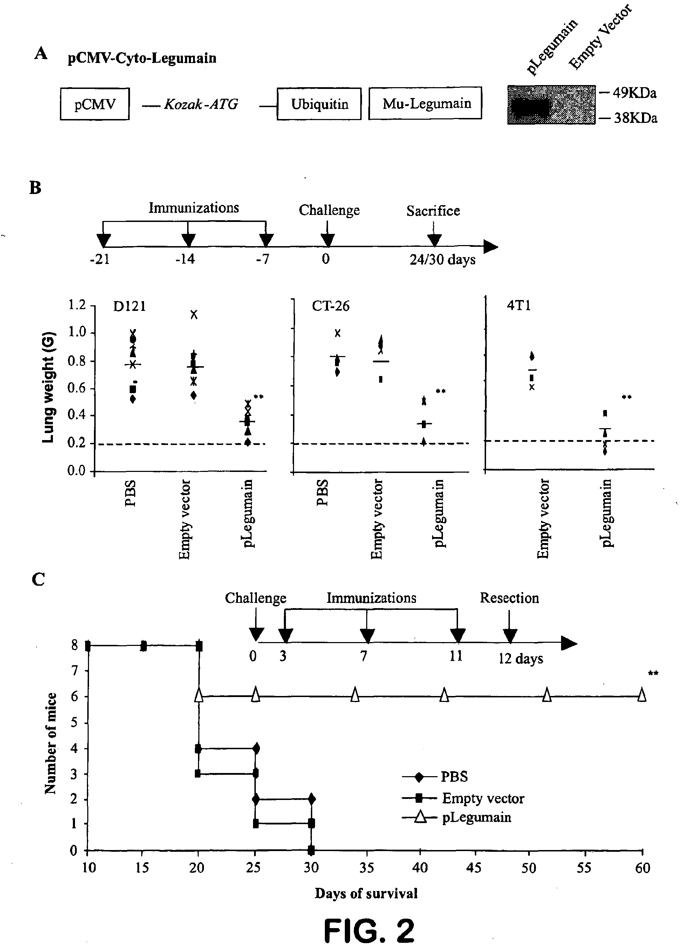

FIG. 2. Targeting of legumain expressing cells results in suppression of tumor progression. (A) Schematic of a DNA composition of the invention constructed with the pCMV/myc/cyto vector backbone where the legumain gene was fused to the C-terminus of mutant polyubiquitin. The entire fragment was inserted and protein expression was demonstrated by Western blotting. (B) Prophylactic model: the vaccination schedule was designed for three immunizations at 1-week intervals followed by an i.v. challenge with about 2×10⁵ D121 non-small cell lung cancer cells, about 5x10⁴ CT26 colon cancer cells, and mammary fat pad injection with about 7 x 10³ 4T1 breast cancer cells. Lung weights were determined 24 days (D121 or CT-26) or 30 days (4T1) after tumor cell challenge and analyzed in each group. Differences between the two control groups (PBS and /or empty vector) and the treatment group were statistically significant **P<0.005. Normal lung weight =0.2g. (C) Therapeutic model: groups of BALB/c mice (n=8) were initially injected into the mammary fat pad with about 7 x 10³ 4T1 breast cancer cells and thereafter vaccinated three times on days 3, 7 and 11 with PBS, empty vector or the pLegumain DNA composition, respectively, and primary tumors excised on day 12. Survival plots represent results for 8 mice in each of the treatment and control groups. Difference between the empty vector control group and the treatment group was statistically significant **P<0.005.

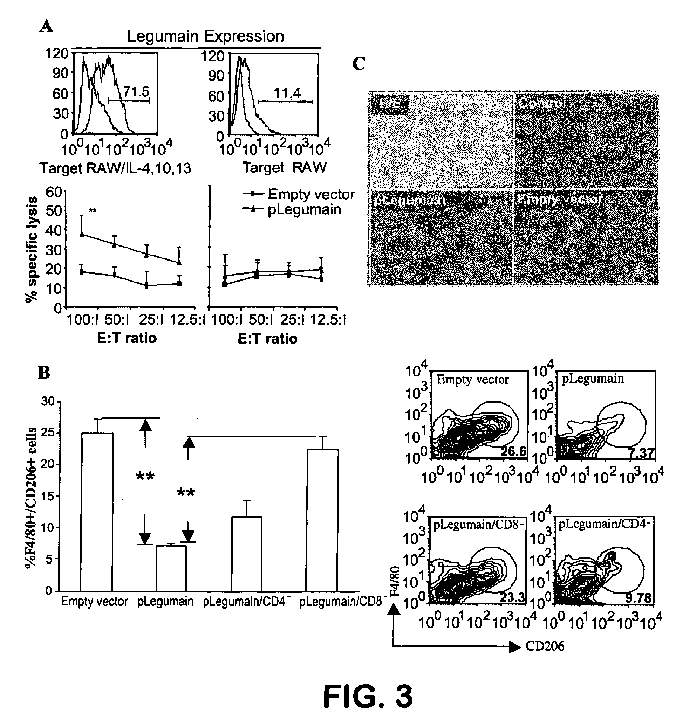

FIG. 3. TAM population in the tumor stroma is decreased by CD8⁺ specific CTLs induced by the legumain-based DNA composition. (A) RAW macrophage cells highly express legumain after culturing with 10 ng/ml IL-4, IL-10, IL-13 and serve as target cells in a 4-hour ⁵¹Cr-release assay. Splenocytes isolated from mice immunized with the pLegumain composition effectively killed RAW cells treated with these cytokines *in vitro* at different effector-to-target cell ratios, but failed to induce cytotoxic killing of unstimulated RAW cells lacking legumain expression. **P<0.005 compared to control groups. (B) Flow cytometry detects the percentage of TAM populations with specific macrophage markers (CD206 and F4/80) in tumor tissue after vaccination. The percentage of TAM populations among tumor tissue cells, isolated from mice treated with the DNA composition of the invention was shown to be reduced. There was no decrease in TAM populations isolated from mice treated with either empty vector or pLegumain following CD8⁺ T cell depletion (**P<0.005). (C) The results of flow cytometry were confirmed by immunohistochemical staining evaluated by confocal microscopy. The population of TAMs in the tumor stroma was dramatically decreased after vaccination. Magnification x5 (H/E), x35 (Control, Empty Vector and pLegumain).

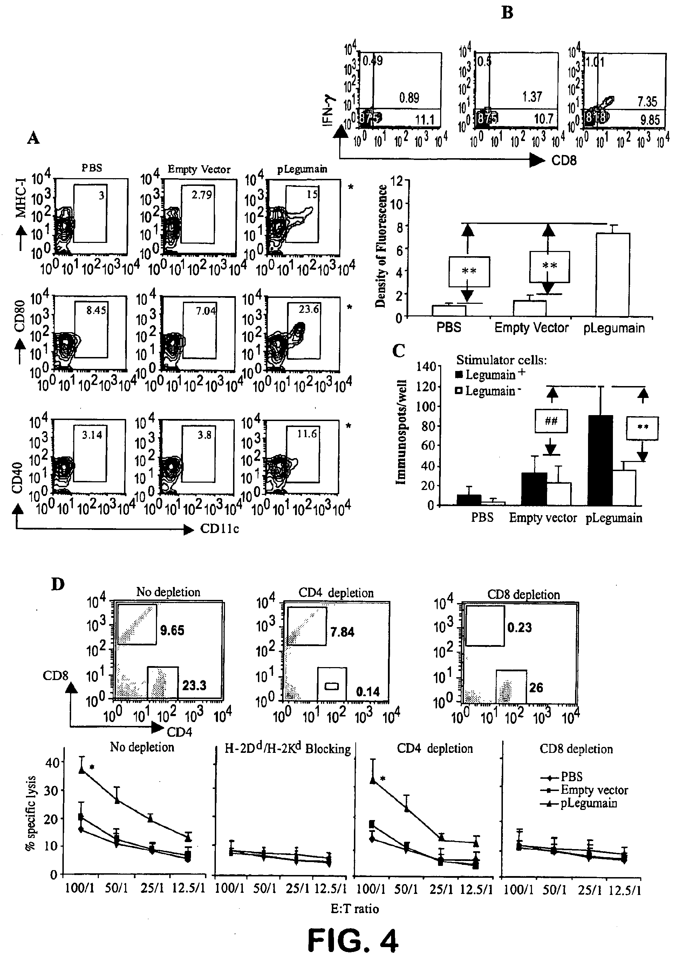

FIG. 4. MHC-class I antigen restricted specific CD8⁺ T cell response against legumain expressing cells. (A) FACS plots show that DNA treatment enhances expression of costimulatory molecules by DCs. Lymphocytes from Peyer's patches obtained 3 days after vaccination were stained with FITC labeled anti-CD11cAb in combination with PE conjugated anti-CD80 Ab, anti-MHC class I or anti-CD40 Abs. (*P<0.05, compared to control groups). (B) Intracytoplasmic INF-gamma release of CD8⁺ T cells was measured by FACS analysis. **P<0.005, compared to control groups. (C) Production of specific INF-gamma was verified at the single cell level by ELISPOT. This is indicated for lymphocytes from immunized mice restimulated with either legumain⁺ 4T1 tumor tissue cells or legumain⁻ 4T1 cells, as depicted by the number of immunospots formed per well. **P<0.005 compared to treatment group without stimulation. ##P<0.005 compared to control groups. (D) Splenocytes isolated from treated mice were effective in killing TAMs by using a ⁵¹Cr release assay (*P<0.01, compared to control groups). Inhibition experiments with Abs against H2Kd/H-2Dd MHC class I antigens showed that T cell mediated tumor cell lysis was MHC class I Ag restricted. *In vivo* depletions of CD4⁺ or CD8⁺ T cells indicated that lymphocytes isolated from vaccinated mice, which were thereafter depleted of CD8⁺ T cells, failed to induce cytotoxic killing of target cells, Depletion of CD4⁺ T cells did not abrogate cytotoxic killing of these same target cells. *P<0.01 compared to PBS or empty vector group.

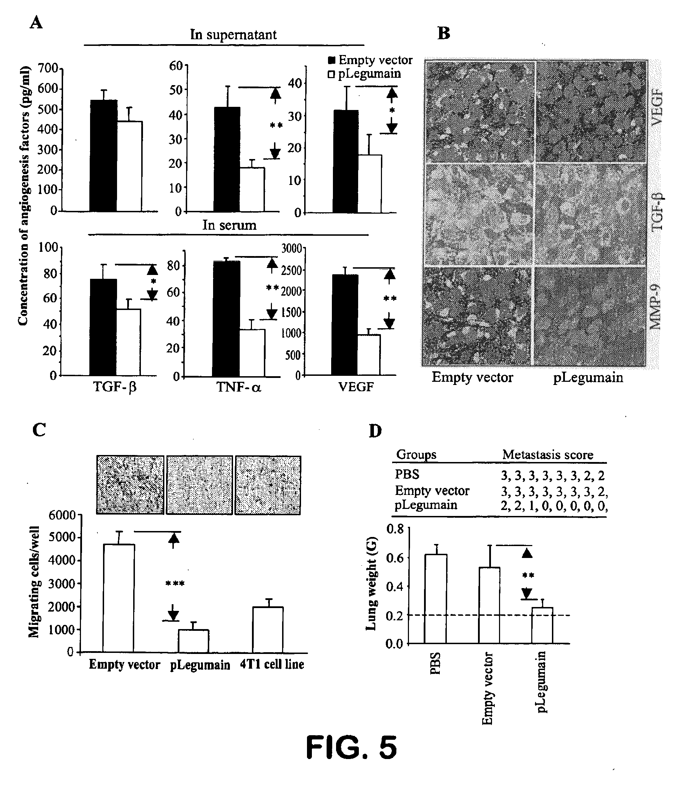

FIG. 5. Abrogation of TAMs results in decreases of growth factors release, tumor cell migration and metastases. (A) The DNA composition of the invention decreased the release of growth factors by TAMs. 4T1 breast tumor tissue and mouse serum were harvested 12 days after vaccinations and tumor cell challenge. After 24 hours or 48 hours culturing, the supernatants of tumor tissue cells were harvested, and the concentrations of TGF-beta , TNF-alpha and VEGF in serum or supernatants measured by ELISA. There were significant differences between the treatment group and control groups. *P<0.01, **P<0.005. (B) Immunohistochemical staining was performed to determine expression of these growth factors in the tumor microenvironment. The vaccine treatment groups showed that VEGF, TGF-beta and MMP-9 releases were decreased after a reduction in TAMs, compared with the empty vector groups. (C) A transwell migration assay was performed to determine tumor cell migration after vaccination. The number of migrating cells was markedly reduced after vaccination. ***P<0.001 compared to the empty vector group. (D) *In vivo* experiments were performed to determine the ability of mice to form tumor metastases. The mice were treated with the vaccine within the therapeutic setting as described above. Tumor metastasis scores and lung weights were measured 25 days after primary tumor excision. The metastasis scores are expressed as the % lung surface covered by fused metastatic foci: 0=none; 1= <5%, 2=5% to 50%, and 3= >50%. Differences in lung weights between the group of mice treated with vaccine and all control groups were statistically significant (**P<0.005).

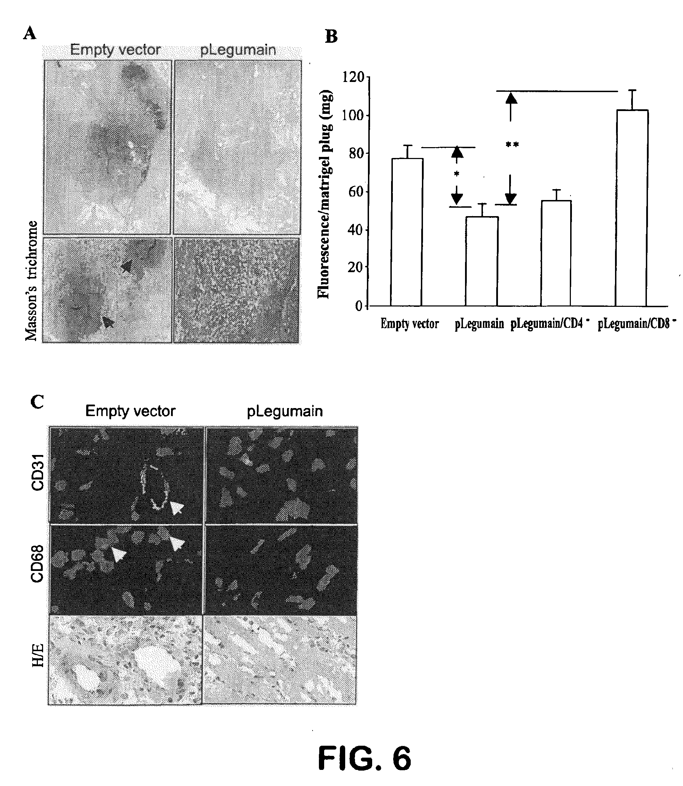

FIG. 6. Elimination of TAMs results in a reduction of tumor angiogenesis. Suppression of VEGF-induced angiogenesis: BALB/c mice were vaccinated with *S. typhimurium* transfected with either empty vector, pLegumain, or pLegumain after either CD8⁺ or CD4⁺ T cell depletion *in vivo,* respectively. One week after the last vaccination, Matrigel was implanted s.c. into the midline of the abdomen of mice. Vascularization was induced by VEGF or bFGF. (A) The images were taken by a digital camera 6 days after Matrigel plug implantation. Additionally, the section of Matrigel plugs stained with Massion's trichrome indicate blood vessel growth in Matrigel plugs as highlighted by arrows (Magnification x5). (B) Quantification of vessel growth was performed after *in vivo* staining of endothelium with FITC-labeled isolectin B4 and evaluation by fluorimetry. There was a decrease in the VEGF-induced neovasculature only after vaccination with the vector encoding legumain but not after vaccination with the empty vector or with pLegumain after depletion of CD8⁺ T cells. **P<0.005, *P<0.01 compared to the legumain treatment group. (C) Immunohistochemical staining was performed and evaluated by confocal microscopy. The cross sections of matrigel plugs were stained to determine the cell type that grew in or migrated into these plugs. The images indicate that endothelial cells with the CD31 marker or macrophages with the CD68 marker grew in or migrated into Matrigel plugs as indicated by arrows (Magnification x35). H/E staining served as a control (Magnification x5).

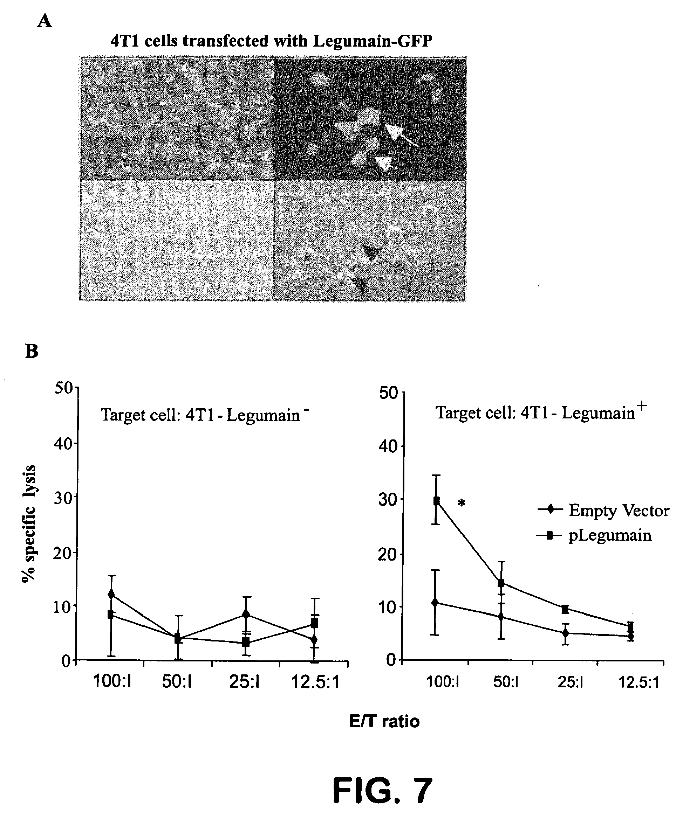

FIG. 7. The 4T1 cell line was stably transfected by a retrovirus harboring the legumain plasmid and then used as target cells for splenocytes from immunized mice (Panel A, left photos have a 5x magnification; right photos have a 35x magnification), the images were taken 2 days after transfection and the positive cells are indicated by arrows. The ⁵¹Cr release assay data are shown in Panel B. The splenocytes isolated from mice immunized with the pLegumain DNA composition were effective in killing 4T1 cells transfected with legumain (*P<0.01, compared to empty vector control groups). The tumor specific T cell-mediated killing was specific for legumain since normal 4T1 cells, lacking in legumain expression, were not lysed.
FIG. 8 shows the nucleotide sequence of a nucleic acid encoding human legumain (SEQ ID NO: 1).
FIG. 9 shows the amino acid residue sequence (SEQ ID NO: 2) of human legumain.
FIG. 10 shows the nucleotide sequence (SEQ ID NO: 3) of a nucleic acid encoding murine legumain.
FIG. 11 shows the amino acid residue sequence (SEQ ID NO: 4) of murine legumain.
FIG. 12 shows the nucleotide sequence (SEQ ID NO: 5) of a nucleic acid encoding human IL-2.
FIG. 13 shows the amino acid residue sequence of human IL-2 (SEQ ID NO: 6).
FIG. 14 shows the nucleotide sequence (SEQ ID NO: 7) of a nucleic acid encoding human CCL21.
FIG. 15 shows the amino acid residue sequence of human CCL21 (SEQ ID NO: 8).
FIG. 16 shows the nucleotide sequence (SEQ ID NO: 9) of a nucleic acid encoding human CD40L.
FIG. 17 shows the amino acid residue sequence of human CD40L (SEQ ID NO: 10).
FIG. 18 shows the nucleotide sequence of ubiquinated murine legumain (SEQ ID NO: 11).
FIG. 19 shows the amino acid residue sequence of ubiquinated murine legumain (SEQ ID NO: 12).
FIG. 20 shows the amino acid residue sequence of murine legumain epitope sequences.
FIG. 21 shows a schematic representation of plasmids encoding legumain minigenes comprising immunogenic fragments of legumain.

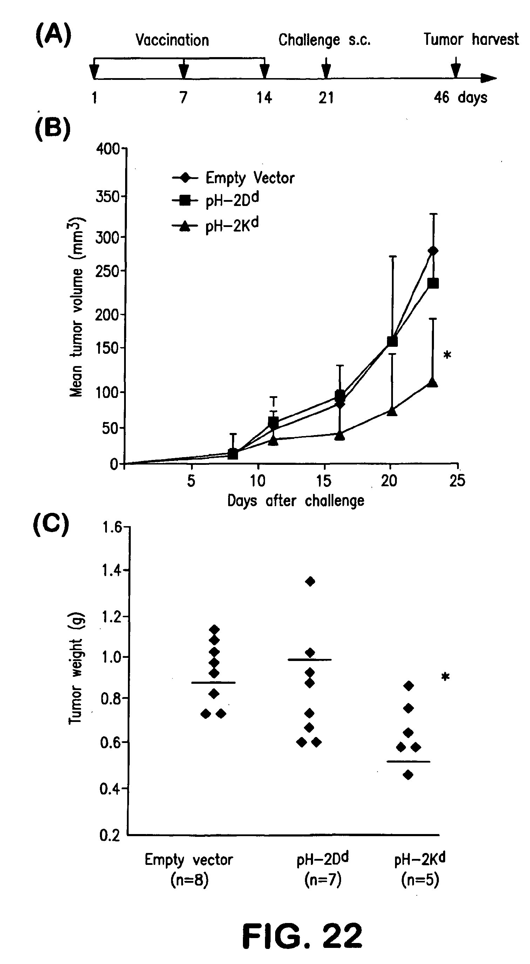

FIG. 22 demonstrates that the pCMV-Kb/Kd minigene composition protects mice from challenge with D2F2 breast carcinoma cells. Groups of BALB/c mice (n=8) were immunized 3 times at 1-week intervals with doubly attenuated *Salmonella typhimurium* RE-88 harboring the vectors indicated. Mice were challenged 1 week after the last immunization by i.v., injection of 2x10⁵ D2F2 breast carcinoma cells. A. Schematic of experimental protocol. B. Mean tumor volume of mice 5 to 25 days after tumor cell challenge. C. Tumor weight of mice 25 days after cell challenge, *, *P<0.05* compared to empty vector control group.

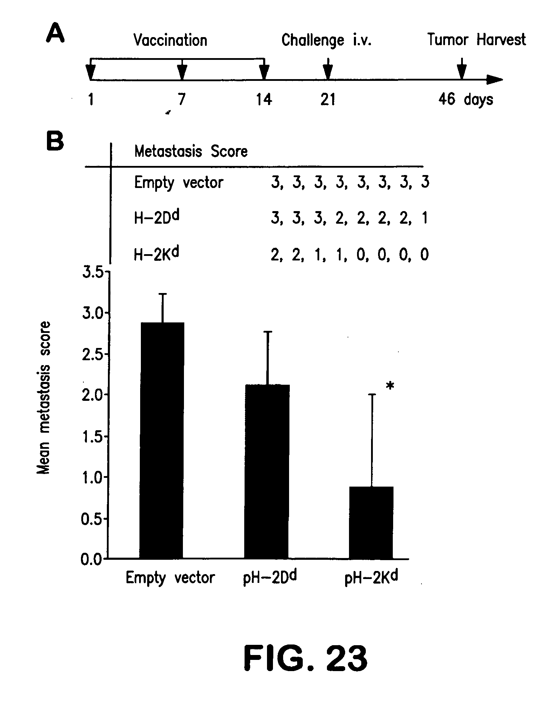

FIG. 23 demonstrates that the pKd minigene vaccine prevents D2F2 breast carcinoma metastasis in syngeneic BALB/c mice. Groups of mice (n=8) were immunized 3 times at 1-week intervals by gavage with attenuated *Salmonella typhimurium* RE-88 harboring the vectors indicated. Mice were challenged 2 weeks after the last immunization by i.v., injection of 1x10⁵ D2F2 breast carcinoma cells. A. Schematic of experimental protocol. B. Average lung metastasis score of mice from each experimental group 25 days after tumor cell challenge. Tumor metastasis scores on lungs were established by estimating the % of surface area covered by fused metastases as follows: 0, no metastases; 1, <20%; 2, 20% to 50% and 3, >50% represented by individual symbols for each treatment group. * *P<0.05* compared to empty vector control group.

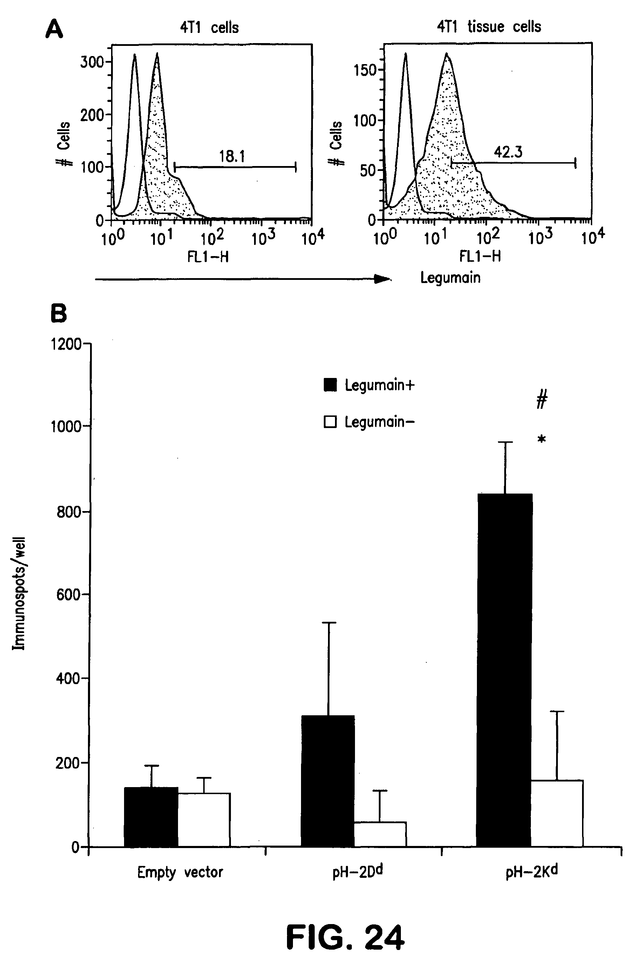

FIG. 24 illustrates IFN-γ release in legumain-specific T cells induced by the pCMV-Kb/Kd minigene composition. A. The expression of legumain by 4T1 cells freshly harvested from tumor tissues were used as stimulator cells. Flow cytometry was performed to indicate the extent of legumain expression on those cells. B. Production of IFN-γ was verified at the single cell level by ELISPOT as lymphocytes from immunized mice were restimulated with either legumain ⁺ 4T1 tumor cells freshly harvested from tumor tissue or legumain⁻ 4T1 tissue culture cells. IFN-γ release is indicated by the number of immunospots formed per well. * *P<0.05,* compared with groups of mice whose lymphocytes were not stimulated by legumain⁺ tumor cells. #*P<0.05,* compared with control groups.

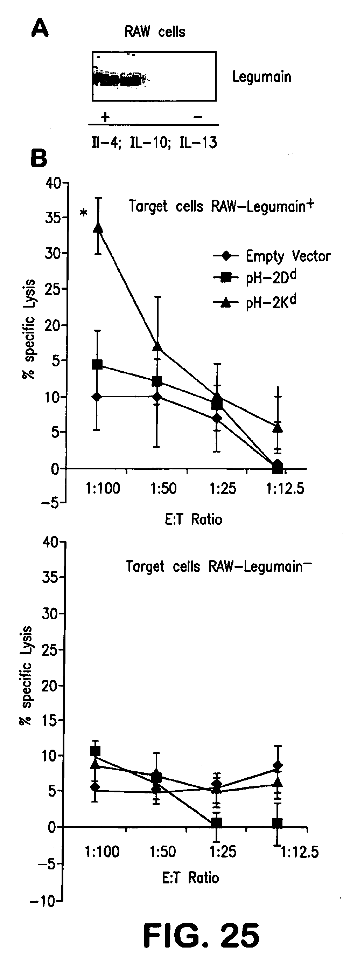

FIG. 25 illustrates the specific CTL killing of legumain positive macrophage cells induced by the pCMV-Kb/Kd minigene composition. A. The expression of legumain by the macrophage cell line RAW is indicated after culture with IL-4, IL-10 and IL-13. Western blot analysis was performed to indicate legumain expression on those cells. B. Groups of immunized BALB/c mice (n=4) were sacrificed 2 weeks after the last immunization and splenocytes isolated from them were stimulated with irradiated 4T1 legumain⁺ cells for 5 days. Thereafter, cytotoxicity assays were performed with either wild type RAW legumain⁻ cells (lower panel), or RAW legumain⁺ cells as target cells (upper panel). * *P<0.05,* compared to empty vector control group while using RAW legumain⁺ cells as target cells.

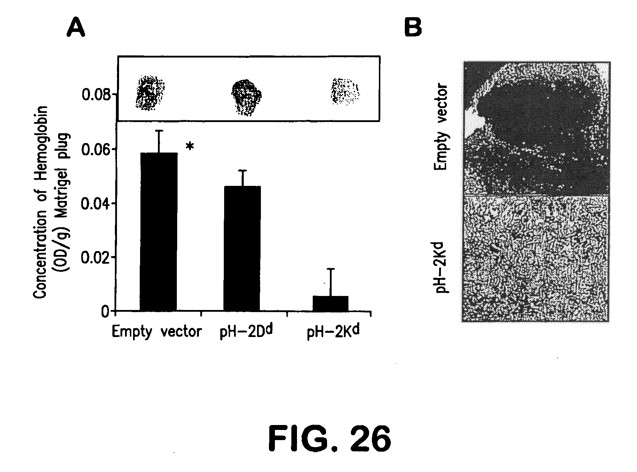

FIG. 26 illustrates suppression of angiogenesis in syngeneic BALB/c mice induced by the pCMV-Kb/Kd minigene composition. A. Result of Matrigel assay. Matrigel was implanted into mice vaccinated with either empty vector, pCMV-Db/Dd or pCMV-Kb/Kd vaccines. The measurement of hemoglobin (Hb) concentration in Matrigel plugs was performed for quantification of blood vessel growth. The average Hb concentration of Matrigel plugs from each group of mice is depicted by the bar graph (n=4; mean + SD). *, *P<0.05*, compared to empty vector control group. B. Masson's trichrome staining of Matrigel sections prepared 7 days after Matrigel plug implantation. Arrows indicate blood vessels in the Matrigel plug.

### DETAILED DESCRIPTION OF PREFERRED EMBODIMENTS

The present invention provides a DNA composition, which targets the tumor-associated cells such as tumor-associated macrophages (TAMs). The DNA composition comprises a DNA minigene construct that encodes an immunogenic polypeptide comprising a plurality of legumain fragments, each of which is preceded by the three amino acids AAY, the polypeptide being expressed in tumor-associated cells, the immunogenic fragments being joined together serially by said three amino acids AAY between each successive fragment in the polypeptide, wherein the polypeptide is capable of eliciting an immune response against the tumor-associated cells, and is expressible in immune cells, and wherein the DNA minigene construct is incorporated in a pharmaceutically acceptable carrier, wherein said immunogenic fragments have an amino acid sequence according to SEQ ID NOs: 16, 17 and 18.

The term "DNA construct" as used herein and in the appended claims refers to a DNA structure that encodes for a protein or polypeptide of interest, such as legumain or an immunogenic legumain fragment (epitope), collectively referred to as "legumain DNA", as well as proteins, such as IL-2, CCL21, CD40L and the like. Preferably, each immunogenic fragment comprises about 8 to 10 amino acid residues in length. DNA constructs include any DNA that can be transcribed in target cells, including linear DNA and plasmid DNA, as well as DNA which has been incorporated into the genetic material of a cell or virus. Preferably, the DNA construct is a DNA that has been incorporated in a viral or bacterial delivery vector, e.g., an attenuated viral or bacterial vector that is non-pathogenic. When a subject is treated with a composition of the invention, the legumain DNA is delivered to immune cells (e.g., macrophages and dendritic cells), which then express the protein or polypeptide including an immunogenic fragment thereof. Viral and bacterial carriers of the legumain DNA do not themselves express legumain.

The DNA construct described herein is a minigene construct that encodes an immunogenic polypeptide comprising a plurality (e.g., 2 to 5) of immunogenic fragments of an endopeptidase that is highly expressed in TAMs (e.g., legumain). As used herein, the term "minigene" refers to DNA constructs that encode multiple portions (fragments) of a protein of interest, which are linked together, preferably by small peptides of at least three amino acids. Thus minigenes encode polypeptides that include immunogenic portions of the protein of interest, but do not encode the entire protein of interest. Preferably, a minigene construct encodes a polypeptide that comprises about 2 to 5 immunogenic fragments (i.e., peptide sequences from epitope regions of the protein), preferably joined together by linking peptides. The minigene can also include sequences that encode leader sequences and/or other sequences that are useful for facilitating expression or transport of the polypeptide.

As noted above, the immunogenic fragments in the polypeptide preferably are joined together by a linker or spacer peptide between each fragment. The linker peptide preferably comprises at least three amino acid residues (e.g., AAA or AAY). Preferably, the DNA construct encoding an immunogenic fragment of legumain also encodes a leader sequence, such as an endoplasmic reticulum (ER) leader sequence, connected to the N-terminus of the polypeptide encoded by the DNA construct by a linker peptide. When the DNA construct encodes a polypeptide comprising two or more immunogenic legumain fragments, the leader sequence preferably is linked to the first immunogenic fragment from the N-terminus of thereof. Preferably, immunogenic fragments of the endopeptidase consist of about 8 to 10 contiguous amino acid residues from one or more epitope region thereof.

Immunogenic fragments of endopeptidases such as legumain, including human legumain, can be identified by methods well known in the art, such as the HLA Binding Predictions program provided by the Bioinformatics & Molecular Analysis Section (BIMAS) of the National Institutes of Health (NIH) at the NIH www website.

The DNA compositions of the present invention stimulate formation of CTLs that are active against tumor-associated macrophages that express the endopeptidase. Such tumor-associated macrophages are selectively targeted by CTLs that are produced in response to immunization by the DNA compositions of the invention. Elimination or abrogation of TAMs changes the tumor microenvironment, resulting in suppression of tumor growth and tumor metastases.

As used herein, the term "immunity" refers to long term immunological protection against the virulent form of the infectious agent or tumor antigen. The term "immunization" refers to exposure to an antigen of a pathogenic agent derived from a non-virulent source, which results in immunity to the pathogen in the treated subject.

A DNA construct useful in a DNA composition described herein preferably comprises a nucleic acid that encodes a polypeptide comprising legumain (e.g., human legumain) or immunogenic fragments of legumain, and which is operably linked to regulatory elements needed for gene expression in immune cells. In a preferred embodiment, the DNA construct encodes for full length human legumain protein (SEQ ID NO: 2) or a polypeptide sharing a high degree of homology of at least about 80 % therewith (e.g., porcine legumain, mouse legumain, ant the like), or an immunogenic fragment thereof, and which can elicit an immune response against cells that overexpress legumain.

In a particularly preferred embodiment, the DNA construct comprises a minigene encoding 2 to 5 immunogenic fragments of legumain (e.g., human legumain) linked by three-amino acid linker peptides (e.g., AAA or AAY), with one linker peptide between each immunogenic fragment.

Useful DNA constructs, including minigene constructs, preferably include regulatory elements necessary for expression of nucleotides. Such elements include, for example, a promoter, an initiation codon, a stop codon, and a polyadenylation signal. In addition, enhancers are often required for expression of a sequence that encodes an immunogenic target protein. As is known in the art, these elements are preferably operably linked to the sequence that encodes the desired protein. Regulatory elements are preferably selected that are operable in the species to which they are to be administered. Preferably, the DNA construct is in the form of a plasmid or is incorporated into a viral or bacterial vector. The DNA construct encoding legumain initially can be incorporated into a bacterial vector by transfection, using methods well known in the art. Subsequently, the transformed bacteria can be cultured to provide a ready stock of bacteria, which include legumain DNA within the genetic material of the bacteria. Cultures of such transformed bacteria provide a ready source for the DNA compositions of the present invention.

Initiation codons and stop codons are preferably included as part of a nucleotide sequence that encodes the endopeptidase or immunogenic polypeptide in a DNA composition of the present invention. The initiation and termination codons must be in frame with the coding sequence.

Promoters and polyadenylation signals included in a composition of the present invention preferably are selected to be functional within the cells of the subject to be immunized.

Examples of promoters useful in the compositions of the present invention, especially in the production of a genetic vaccine DNA composition for humans, include but are not limited to promoters from Simian Virus 40 (SV40), Mouse Mammary Tumor Virus (MMTV) promoter, Human Immunodeficiency Virus (HIV) such as the HIV Long Terminal Repeat (LTR) promoter, Moloney virus, Cytomegalovirus (CMV) such as the CMV immediate early promoter, Epstein Barr Virus (EBV), Rous Sarcoma Virus (RSV) as well as promoters from human genes, such as human actin, human myosin, human hemoglobin, human muscle creatine, and human metalothionein.

Examples of polyadenylation signals useful in the DNA compositions of the present invention, especially in the production of a DNA composition for humans, include but are not limited to SV40 polyadenylation signals and LTR polyadenylation signals.

In addition to the regulatory elements required for DNA expression, other elements can also be included in the DNA molecule. Such additional elements include enhancers. The enhancer can be, for example, human actin, human myosin, human hemoglobin, human muscle creatine and viral enhancers such as those from CMV, RSV and EBV.

Regulatory sequences and codons are generally species dependent, so in order to maximize protein production, the regulatory sequences and codons are preferably selected to be effective in the species to be immunized. One having ordinary skill in the art can produce DNA constructs that are functional in a given subject species.

DNA constructs useful in the present compositions can be "naked" DNA, as defined in Restifo et al. Gene Therapy 2000; 7:89-92. Preferably, the DNA construct is in the form of a plasmid or DNA that is incorporated into the genetic material of an attenuated virus or attenuated bacterium. Useful delivery vehicles or carriers include biodegradable microcapsules, immuno-stimulating complexes (ISCOMs), and liposomes for naked DNA constructs, and various physiologically acceptable buffers for genetically engineered attenuated live viruses or bacteria.

Examples of suitable attenuated live bacterial vectors that can be transformed to incorporate a FAP DNA construct include *Salmonella typhimurium, Salmonella typhi, Shigella* species, *Bacillus* species, *Lactobacillus* species, *Bacille Calmette-Guerin (BCG), Escherichia coli, Vibrio cholerae, Campylobacter* species, *Listeria* species, or any other suitable bacterial vector, as is known in the art. Preferably the vector is an attenuated live *Salmonella typhimurium* vector particularly when the composition is intended for oral administration. Preferred attenuated live *Salmonella typhimurium* include *AroA⁻* strains such as SL7207, or doubly attenuated *AroA*⁻, *dam -* strains, such as RE88.

Methods of transforming live bacterial vectors with an exogenous DNA construct are well described in the art. See, for example, Joseph Sambrook and David W. Russell, Molecular Cloning, A Laboratory Manual, 3rd Ed., Cold Spring Harbor Laboratory Press, Cold Spring Harbor, New York (2001) (Sambrook and Russell). After transformation, the exogenous genetic material is incorporated into the genetic material of the bacterium, so that as the bacteria reproduce, the exogenous DNA is replicated along with the native DNA of the organism. Thus, once the bacterium has been transformed, the normal reproductive processes of the organism provides a ready supply of the exogenous DNA.

Preferred viral vectors include Bacteriophages, Herpes virus, Adenovirus, Adeno-associated virus, Sindbis virus, Polio virus, Vaccinia virus, and Avipox. Methods of transforming viral vector with an exogenous DNA construct are also well described in the art. *See* Sambrook and Russell, above.

Useful liposome carrier vehicles are unilamellar or multilamellar vesicles, having a membrane portion formed of lipophilic material and an interior aqueous portion. The aqueous portion is used to contain the polynucleotide material to be delivered to the target cell. It is generally preferred that the liposome forming materials have a cationic group, such as a quaternary ammonium group, and one or more lipophilic groups, such as saturated or unsaturated alkyl groups having about 6 to about 30 carbon atoms. One group of suitable materials is described in European Patent Publication No. 0187702, and further discussed in U.S. Patent No. 6,228,844 to Wolff et al.*.* Many other suitable liposome-forming cationic lipid compounds are described in the literature. See, e.g., L. Stamatatos, et al., Biochemistry 1988; 27:3917-3925; and H. Eibl, et al., Biophysical Chemistry 1979; 10:261-271. Alternatively, a microsphere such as a polylactide-coglycolide biodegradable microsphere can be utilized. A nucleic acid construct is encapsulated or otherwise complexed with the liposome or microsphere for delivery of the nucleic acid to a tissue, as is known in the art.

Other useful carrier vehicles include polymeric microspheres comprising biodegradable poly(ortho ester) materials, as described by Wang et al., Nat. Mater., 2004; 3(3):190-6. Epub 2004 Feb. 15.

Preferably, the DNA compositions comprise DNA constructs comprising immunogenic fragments of human legumain or a functional homolog thereof. Functional homologs of legumain preferably share at least about 80% amino acid residue sequence identity with human legumain, more preferably, at least about 90%, most preferably at least about 95% identity with human legumain.

GenBank is a genetic sequence database of the National Institutes of Health (NIH), which is an annotated collection of all publicly available DNA sequences. GenBank is part of the International Nucleotide Sequence Database Collaboration, a combined effort of the DNA DataBank of Japan (DDBJ), the European Molecular Biology Laboratory (EMBL), and GenBank at the National Center for Biotechnology Information.

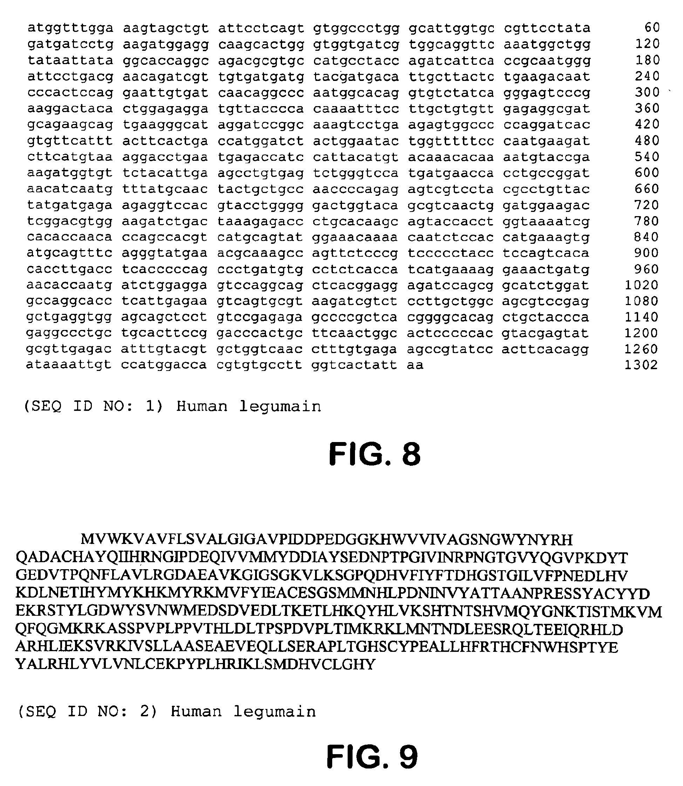

The nucleic acid sequence of a nucleic acid encoding human legumain, SEQ ID NO: 1 (FIG. 8) has been published in GenBank Accession No. BC026250. The corresponding amino acid residue sequence of human legumain is SEQ ID NO: 2 (FIG. 9).

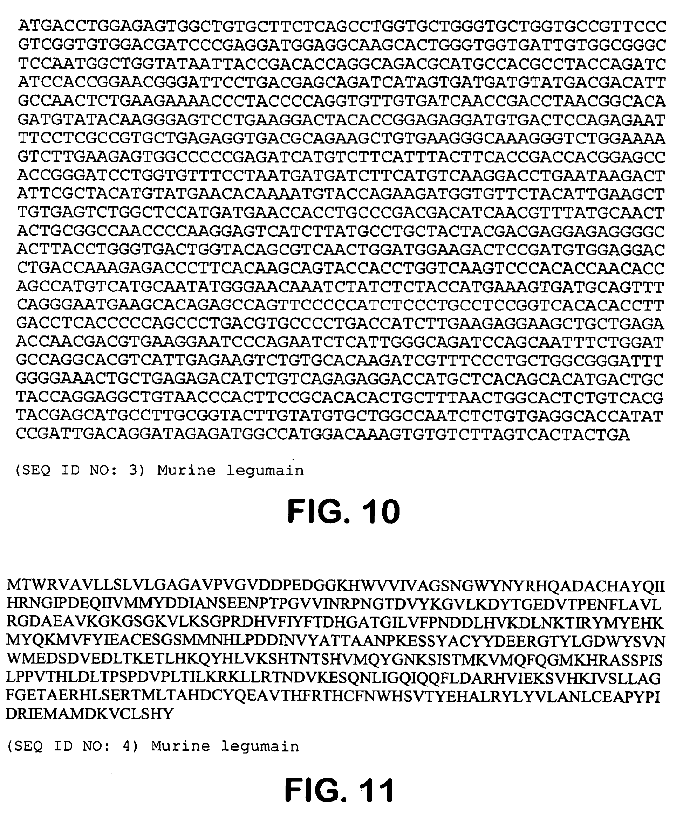

The nucleic acid sequence of a DNA encoding murine legumain, SEQ ID NO: 3 is shown in FIG. 10. The corresponding amino acid residue sequence of murine legumain is SEQ ID NO: 4 (FIG. 11).

Chen et al., J. Biological Chem. 1997, 272(12): 8090-8098, have reported the structure and characterization of porcine legumain, which has about 83 percent sequence identity to human legumain.

Due to the inherent degeneracy of the genetic code, other DNA sequences that encode the amino acid sequence to human legumain, can be used in the practice of the invention. Such DNA sequences include those which are capable of hybridizing to human legumain as well.

DNA sequences that encode human legumain, and which can be used in accordance with the invention, include nucleic acids having deletions, additions or substitutions of different nucleotide residues for those in SEQ ID NO: 1, which result in a sequence that encodes the same legumain gene product. DNA molecules encoding for functionally equivalent homologs of human legumain can also be used in the DNA compositions of the present invention.

The gene product encoded by nucleic acid may also contain deletions, additions or substitutions of amino acid residues within the legumain amino acid residue sequence, which results in a silent change, thus producing a functionally equivalent legumain. Such amino acid substitutions may be made on the basis of similarity in polarity, charge, solubility, hydrophobicity, hydrophilicity, and/or the amphipathic nature of the residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, alanine; asparagine, glutamine; serine, threonine; phenylalanine, tyrosine. As used herein, a functionally equivalent legumain refers to protein that includes one or more epitopes, which when recognized by T cells, allow these same T cells to recognize legumain epitopes displayed on legumain-expressing cells. In preferred embodiments, a functionally equivalent legumain has an amino acid residue sequence that has at least about 80% sequence identity to the amino acid residue sequence of human legumain (SEQ ID NO: 2), e.g., at least about 90% sequence identity, or at least about 95% sequence identity.

The DNA constructs encoding legumain can be engineered in order to alter the legumain coding sequence (relative to the native legumain DNA, SEQ ID NO: 1) for a variety of ends including, but not limited to, alterations that modify processing and expression of the legumain gene product. For example, mutations may be introduced in the DNA using techniques that are well known in the art, e.g., site-directed mutagenesis, to insert new restriction sites, to alter glycosylation patterns, phosphorylation, etc.

In one preferred embodiment, the DNA composition of the invention comprises a DNA construct encoding an immunogenic polypeptide comprising a plurality of immunogenic fragments of human legumain and a DNA construct operably encoding at least one immune effector protein, both of which are expressible in immune cells. As used herein and in the appended claims the phrase "immune effector protein" means a protein that is involved in regulation of an immune system pathway. Preferably, the immune effector protein is a cytokine.

Cytokines are proteins and polypeptides produced by cells that can affect the behavior of other cells, such as cell proliferation, cell differentiation, regulation of immune responses, hematopoiesis, and inflammatory responses. Cytokines have been classified into a number of families, including chemokines, hematopoietins, immunoglobulins, tumor necrosis factors, and a variety of unassigned molecules. *See generally* Oxford Dictionary of Biochemistry and Molecular Biology, Revised Edition, Oxford University Press, 2000; and C. A. Janeway, P. Travers, M. Walport and M. Schlomchik, Immunobiology, Fifth Edition, Garland Publishing, 2001 (hereinafter "Janeway and Travers"). A concise classification of cytokines is presented in Janeway and Travers, Appendix III, pages 677-679.

Hematopoietins include, for example erythropoietin, interleukin-2 (IL-2, a 133 amino acid protein produced by T cells and involved in T cell proliferation), IL-3, IL-4, IL-5, IL-6, IL-7, IL-9, IL-11, IL-13, IL-15 (a 114 amino acid IL-2-like protein, which stimulates the growth of intestinal epithelium, T cells, and NK cells), granulocyte colony-stimulating factor (G-CSF), granulocyte-macrophage colony-stimulating factor (GM-CSF), oncostatin M (OSM), and leukemia inhibitory factor (LIF).

Interferons include, for example, IFN-α, IFN-β, and IFN-γ (a 143 amino acid homodimeric protein produced by T cells and NK cells, which is involved in macrophage activation, increased expression of MHC molecules and antigen processing components, IG class switching, and suppression of T_{H}2).

Immunoglobulins include, for example, B7.1 (CD80), and B7.2 (CD86), both of which co-stimulate T cell responses.

The tumor necrosis factor (TNF) family includes, for example, TNF-α, TNF-β (lymphotoxin), lymphotoxin-β (LT-β), CD40 ligands, Fas ligand, CD27 ligand, CD30 ligand, 4-1BB ligand, Trail, and OPG ligand.

The biological roles of CD40 ligand (CD40L), particularly its interaction with CD40 expressed on antigen presenting cells during costimulation of T cell activation, are well known in the art. CD40 is a 48 kDa glycoprotein expressed on the surface of all mature B cells, most mature B-cell malignancies, and some early B-cell acute lymphocytic leukemias, but it is not expressed on plasma cells, Clark, Tissue Antigens 1990, 35:33-36. CD40L, a type II membrane protein of about 35 kDa, is expressed on the surface of T cells upon antigen recognition. Members of the TNF family are biologically most active when expressed as homotrimers. CD40L is no exception in this regard and can be expressed as a homotrimer (CD40LT) by modification of a 33 amino acid leucine zipper motif fused to the N-terminus of the entire extracellular domain of this ligand. CD40LT DNA has been reported by Gurunathan et al. J. Immunol. 1998, 161:4563, to enhance cellular immune responses such as induction of IFN-γ and cytolytic T cell activity when mice were vaccinated with DNA encoding the highly immunogenic model antigen β-galactosidase.

CD40LT is an important factor in the activation of T cells necessary to induce an effective protective immunity against tumor self-antigens. Once MHC class I antigen:peptide complexes are taken up by dendritic cells (DCs) and presented to naive T cells, the first antigen signal is delivered via T cell receptors (TCR), followed by upregulation of CD40LT. On the T cell surface, CD40LT then induces costimulatory activity on DCs via CD40-CD40LT interactions. Thus primed, these antigen presenting cells can express costimulatory molecules B7.1 (CD80) and B7.2 (CD86), which sends a second costimulatory signal to T cells via interaction with CD28, an event required for full activation of T cells to concurrently produce pro-inflammatory cytokines INF-γ and IL12, and to perform effector functions.

Various cytokines that are not assigned to a particular family include, for example, tumor growth factor-β (TGF-β), IL-1α, IL-1β, IL-1 RA, IL-10, IL-12 (natural killer cell stimulatory factor; a heterodimer having a 197 amino acid chain and a 306 amino acid chain, which is involved in NK cell activation and induction of T cell differentiation to T_{H}1-like cells), macrophage inhibitory factor (MIF), IL-16, IL-17 (a cytokine production-inducing factor, which induces cytokine production in epithelia, endothelia, and fibroblasts), and IL-18.

Chemokines are a family of cytokines that are relatively small chemoattractant proteins and polypeptides, which stimulate the migration and activation of various cells, such as leucocyte migration (e.g., phagocytes and lymphocytes). Chemokines play a role in inflammation and other immune responses. Chemokines have been classified into a number of families, including the C chemokines, CC chemokines, CXC chemokines, and CX₃C chemokines. The names refer to the number and spacing of cysteine (c) residues in the molecules; C chemokines having one cysteine, CC chemokines having two contiguous cysteines, CXC having two cysteines separated by a single amino acid residue, and CX₃C chemokines having two cysteines separated by three amino acid residues. Chemokines interact with a number of chemokine receptors present on cell surfaces. *See* Janeway and Travers, Appendix IV, page 680.

In addition, chemokines can have immunomodulating activity and have been implicated in immune responses to cancer. For example, murine 6Ckine/SLC, the mouse analog of the human secondary lymphoid tissue chemokine (SLC), now commonly referred to as CCL21, has been reported to induce an antitumor response in a C-26 colon carcinoma tumor cell line. *See* Vicari, et al. J. Immunol. 2000; 165(4):1992-2000. Human CCL21 and its murine counterpart, 6Ckine/SLC, are classified as CC chemokines, which interact with the CCR7 chemokine receptor. Murine 6Ckine/SLC (muCCL21) is also reported by Vicari *et al.* to be a ligand for the CXCR3 chemokine receptor. Human CCL21, murine muCCL21 and a variety of other chemokines are implicated in the regulation of various immune system cells such as dendritic cells, T cells, and natural killer (NK) cells.

Mig and IP-10 are CXC chemokines that interact with the CXCR3 receptor, which is associated with activated T cells. Lymphotactin is a C chemokine, which interacts with the XCR1 receptor associated with T cells and NK cells. Fractalkine is a CX₃C chemokine, which interact with the CX₃CR1 receptor that is associated with T cells, monocytes and neutrophils.

Particularly preferred immune effector proteins to be encoded by the DNA compositions of the present invention include cytokines IL-2 (a hematopoietin), CCL21 (a chemokine), as well as CD40 ligands such as CD40 ligand trimer (CD40LT), a TNF family cytokine.

DNA and protein sequences for human IL-2 have been published in GenBank, Accession No. BC070338. The DNA and protein sequences of murine IL-2 have been in GenBank, Accession No. NM 008366.

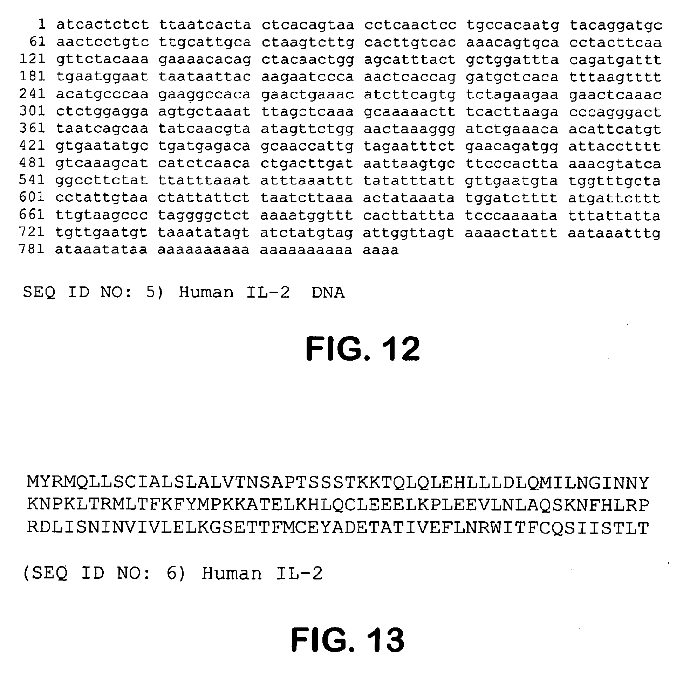

The nucleic acid sequence encoding human IL-2 is presented in FIG. 12 (SEQ ID NO: 5), and its corresponding amino acid residue sequence (SEQ ID NO: 6) is provided in FIG. 13.

DNA and protein sequences for human CCL21 have been published in GenBank, Accession No. AB002409.

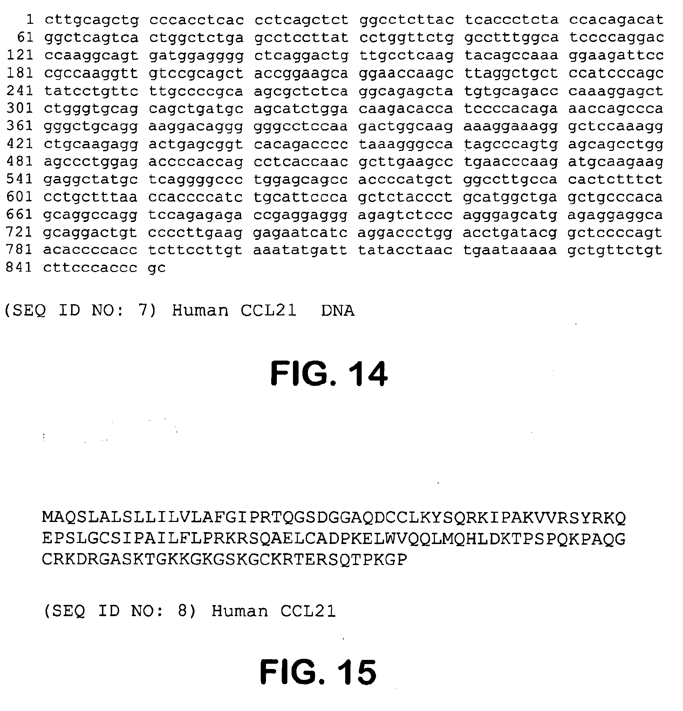

The nucleic acid sequence encoding human CCL21 is presented in FIG. 14 (SEQ ID NO: 7), and its corresponding amino acid residue sequence (SEQ ID NO: 8) is provided in FIG. 15.

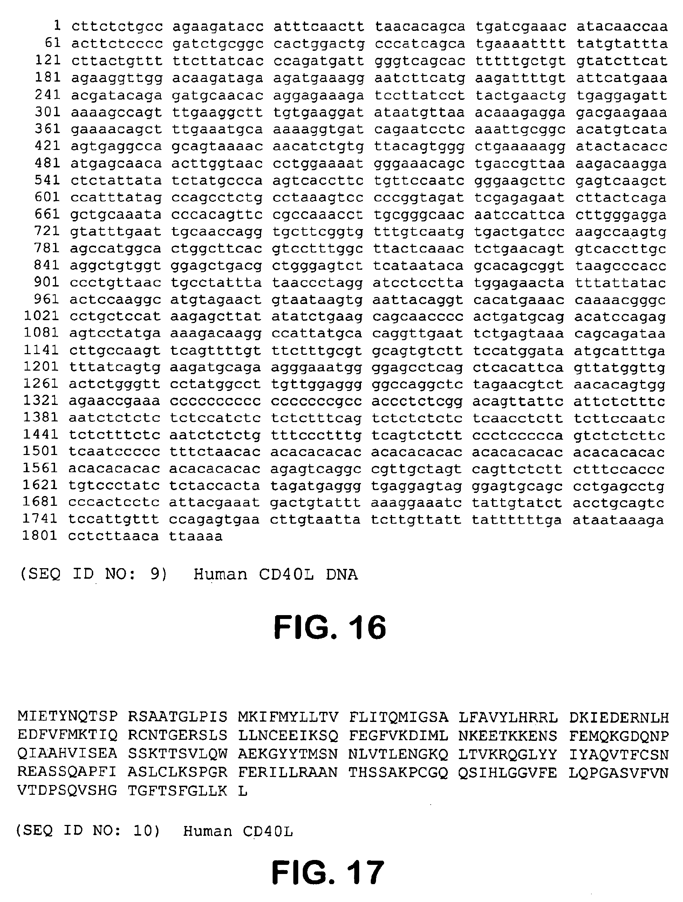

Human CD40 ligand (CD40L) is a 261 amino acid protein, which exists as a trimer (CD40LT) in its most active form. The DNA sequence encoding human CD40L (also known as CD154) has been published in GenBank, Accession No. NM 000074 (FIG. 16, SEQ ID NO: 9). The corresponding protein sequence of CD40L is shown in FIG. 17 (SEQ ID NO: 10).

The use aspects of the present invention involve administering to a mammal a DNA composition comprising a DNA construct encoding legumain, which is expressible in immune cells of the mammal. Preferably, the mammal is a human. The composition can be administered orally, intramuscularly, intranasally, intraperitoneally, subcutaneously, intradermally, or topically, depending upon the particular dosage form in which the composition is prepared. Preferably the composition is prepared in an orally administrable dosage form, such as a solution, suspension, emulsion, capsule, tablet, and the like.

A DNA composition of the invention can be utilized to provide long term inhibition of tumor growth and/or tumor metastases in a patient treated with the composition. The DNA composition can be administered in conjunction with an antitumor chemotherapeutic agent. The DNA composition can be administered together with the chemotherapeutic agent in a combined dosage form, or the composition and chemotherapeutic agent can be administered in separate dosage forms and at separate dosage intervals tailored to the pharmacology of the chemotherapeutic agent being administered.

Chemotherapeutic agents useful in combination with the DNA compositions of the present invention include antitumor agents such as doxorubicin, paclitaxol, a cyclophosphamide, etoposide, 5-fluorouracil, methotrexate, and the like.

DNA compositions of the present invention are preferably formulated with pharmaceutically acceptable carriers or excipients such as water, saline, dextrose, glycerol, and the like, and combinations thereof to aid in formulation and administration of the composition. The compositions can also contain auxiliary substances such as wetting agents, emulsifying agents, buffers, and other auxiliary substances that are well known in the pharmaceutical arts.

The compositions of the present invention are preferably administered orally to a mammal, such as a human, as a solution or suspension in a pharmaceutically acceptable carrier. The compositions can be administered at a DNA concentration in the range of about 1 to about 10 micrograms per milliliter, based on the weight of the DNA that encodes legumain. A particularly preferred dosage form for a DNA composition is a suspension of attenuated legumain-transfected bacteria in a suitable buffer solution, which can be formulated for oral administration. The appropriate dosage of the composition will depend upon the subject to be vaccinated, on the activity of the composition, and in part upon the judgment of the medical practitioner administering or requesting administration of the composition.

The dosage to be administered to the mammal, and the schedule of administration if more than one administration is to be used, will vary from mammal to mammal and upon the dosage form. Effective dosage amounts and administration schedules can be determined empirically through clinical dose-response studies, as is well known in the art. The dosage and dosage schedule is selected to provide a sufficient amount of legumain expression in immune cells to elicit an immune response in the mammal against tumor-associated macrophages that express legumain. Preferably, the dosage of the composition administered to the subject mammal elicits expression of a sufficient amount of legumain antigen in immune cells of the mammal to sustain an immune response against legumain-presenting tumor-associated macrophages that will continue over a period of at least a month, e.g., at least 6 months or at least about one year. In some preferred embodiments, the dosage of legumain transformed cells administered to the patient is about 1 x 10⁸ cells transfected with about 0.3 to about 0.8 µg of legumain DNA.

The compositions of the present invention can be packaged in suitably sterilized containers such as ampules, bottles, or vials, either in multi-dose or in unit dosage forms. The containers are preferably hermetically sealed after being filled with a DNA composition of the invention. Preferably, the compositions are packaged in a container having a label affixed thereto, which label identifies the composition, and bears a notice in a form prescribed by a government agency such as the United States Food and Drug Administration reflecting approval of the composition under appropriate laws, dosage information, and the like. The label preferably contains information about the composition that is useful to an health care professional administering the composition to a patient. The package also preferably contains printed informational materials relating to the administration of the composition, instructions, indications, and any necessary required warnings.

The following examples are provided to further illustrate the features and embodiments of the present invention.

### Materials and Methods.

*Animals, Bacterial Strains and Cell Lines.* Female BALB/c and C57BL/6 mice, 6-8 weeks of age, were purchased from The Scripps Research Institute Rodent Breeding Facility. The double attenuated *S. typhimurium* strain RE88 (*aroA⁻, dam⁻*) was obtained from Remedyne Corporation, Goleta, CA. The murine CT-26 colon cancer cell line was kindly provided by Dr. I. J. Fidler (MD Anderson Cancer Center) and the murine D121 non-small cell lung carcinoma cells were a gift from Dr. L. Eisenbach (Weizmann Institute of Science, Rehovot, Israel). The murine 4T1 breast carcinoma cells were kindly provided by Dr. Suzanne Ostrand-Rosenberg (University of Maryland).

*Immunohistochemical analyses.* Immunohistochemical analyses were performed on 4T1 tumor tissues and Matrigel plug sections. Legumain expression of macrophages was identified on 4T1 tumor tissue sections with biotinylated rat anti-mouse CD68 mAb (BD Bioscience Pharmingen) with GFP-conjugated streptavidin being the secondary reporter reagent. Rabbit anti-legumain antiserum was prepared by immunization with purified human legumain produced in *E. coli.* (Ishii, Methods Enzymol.1994; 244:604-615). The reaction was visualized with Texas-red conjugated streptavidin. Additionally, 4T1 tumor tissue sections and Matrigel plug sections were fixed and stained with MMP-9, VEGF, TGF-beta and F4/80 antibodies (eBioscience, San Diego, CA) in 4T1 tumor tissue section while CD68 and CD31 antibodies (BD Bioscience Pharmingen) were used in Matrigel plug sections. All tissue sections were visualized with Texas red or GFP conjugated streptavidin as the secondary reporting reagent, and the slides analyzed with laser scanning by confocal microscopy (Bio-Rad Laboratories). All the images were captured by a SPOT™ cooled color digital camera system (Diagnostic Instruments. Inc).

*Immunization and Tumor Cell Challenge.* Prophylactic model: BALB/c or C57BL/6 mice were each divided into three experimental groups (n=8) and immunized with PBS, empty vector or pUb-legumain transfected *S. typhimurium.* All mice were challenged by intravenous (i.v.) injection with about 5×10⁴ CT-26 cells (BALB/c), about 2×10⁵ D121 cells (C57BL/6) or mammary gland fat pad injection with about 7×10³ 4T1 cells (BALB/c), 1 week after the last immunization, to induce either experimental or spontaneous pulmonary metastases. The lung weights in experimental and control groups were determined 24 days after tumor cell challenge. Therapeutic model: BALB/c mice were divided into three experimental groups (n=8) and first injected into the fat-pad with about 7×10³ 4T1 cells on day 0 and then immunized 3 times with the DNA composition of the invention starting on days 3, 7 and 11. After 24 days, the primary tumor was excised to determine mouse lung weights and metastasis scores, or mouse survival rates.

*In vivo depletion of CD4⁺ or CD8⁺ T cells, Cytotoxicity and ELISPOT assays.* The depletion of CD4⁺ or CD8⁺ T cells *in vivo* was performed as previously described (Ceredig et al. 1985, Nature 314:98-100). Cytotoxicity was measured and calculated by a standard ⁵¹Cr-release assay as previously reported (Zhou et al. 2005, Blood 106:2026-2032). ELISPOT assays were performed with an ELISPOT kit (BD Bioscience Pharmingen) according to instructions provided by the manufacturer.

*In vivo Matrigel angiogenesis assay.* Matrigel was used for evaluating the suppression of angiogenesis after vaccination. Briefly, BALB/c mice were injected subcutaneously (s.c.) 2 weeks after the last vaccination, into the sternal region with growth factor-reduced Matrigel (BD Bioscience) containing VEGF or bFGF-2 (about 200 ng/plug) and 4T1 tumor cells (about 5x10³/plug) that were previously irradiated with 1000 Gray (about 100,000 rad) of gamma radiation. The endothelium was stained 6 days after Matrigel implantation by i.v. injection with *Bandiera simplofica* lectin I (Isolectin B4), conjugated with fluorescein (Vector Laboratories). This was done along with staining the endothelium of control animals. About 30 minutes later, mice were sacrificed, Matrigel plugs extracted, and fluorescence evaluated by fluorimetry. Additionally, the Matrigel plugs were removed 6 days after Matrigel implantation, fixed in Bouin's solution for 24 hours, and then embedded in paraffin. All tissues were sectioned, mounted onto slides, and stained with Masson's trichrome. All of the images were captured by a SPOT™ cooled color digital camera system as described above.

*Flow cytometry (FACS).* Activation markers of T cells were measured by two-color flow cytometric analysis with a BD Biosciences FACSCALIBUR® analyzer. DC cell markers were determined by staining freshly isolated lymphocytes from successfully vaccinated mice and control mice with anti-CD11c Ab in combination with FITC-conjugated anti-CD40, CD80 and Abs against MHC-Class II Ag. Macrophages bearing high levels of CD206 and F4/80 were quantified by two-color flow analysis. Tumor cells were isolated from successfully vaccinated BALB/c mice and then stained with anti-CD206 Ab conjugated with PE (Cell Science, Inc.), and-F4/80 Ab conjugated with APC and anti-legumain Ab conjugated with FITC, followed by FACS analyses. All antibodies were purchased from Pharmingen, San Diego, CA. IFN-γ release at the intracellular level was determined in lymphocytes of Peyer's patches obtained 3 days after one time immunization, and stained with APC-tagged anti-CD8 Ab. Cells were fixed, permeabilized, and subsequently stained with PE-labeled anti-IFN-γ Ab to detect intracellular expression of IFN-γ.

*Migration Assay.* Cell migration assays were performed by using modified Boyden chambers (Transwell, Coming Inc., Corning, NY). The tumor cells were harvested from tumor tissue of either treated or control groups of mice to perform a trans-well migration assay. After culturing for 4 hours the cells on the lower surface of wells were fixed with 1% paraformaldehyde, stained with 1% crystal violet and counted (Shi et al. 2004, Mol. Cancer Res. 2:395-402).

*Statistical Analysis.* The statistical significance of differential findings between experimental groups and controls was determined by Student's t test. Findings were regarded as significant if two tailed P values were <0.05. Kaplan-Meier analysis was used to evaluate the survival of mice.

### EXAMPLE 1.

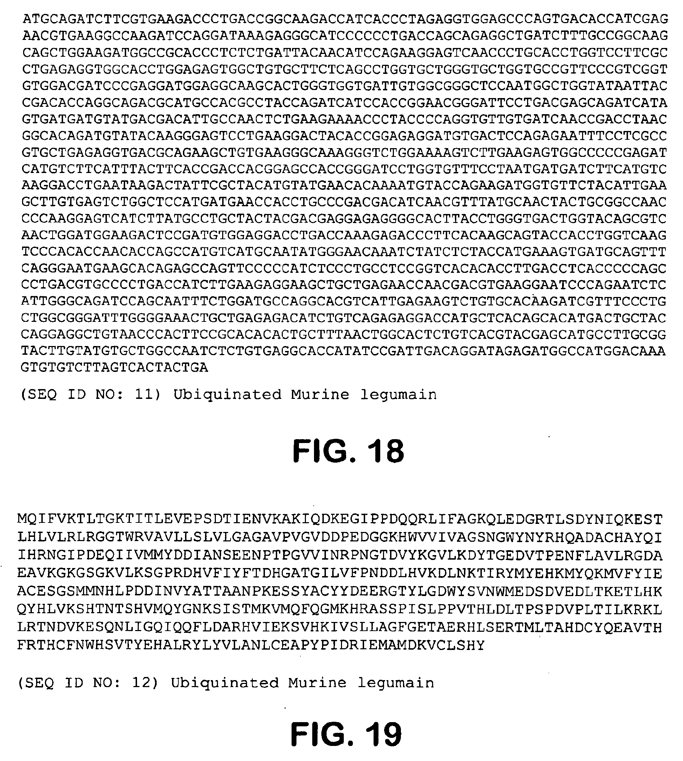

*Vector construction, protein expression and transformation of S. typhimurium with DNA Plasmids.* Two constructs were prepared based on a pCMV vector, which is commercially available from Invitrogen, Carlsbad, CA. The pUb-legumain construct included polyubiquitinated, full-length murine legumain. Full-length legumain murine legumain DNA has the nucleotide sequence shown in FIG. 10, SEQ ID NO: 3 (the amino acid residue sequence of murine legumain is shown in FIG. 11, SEQ ID NO:4). An empty vector construct served as a control. The murine legumain was collected from 4T1 breast cancer cells using total RNA as a template by PCR. An expression vector was established based on the pCMV-cyto vector (Invitrogen) containing the polyubiquitin sequence cloned in front of the legumain sequence. The nucleic acid sequence of the polyubiquinated murine legumain is shown in FIG. 18 (SEQ ID NO: 11). The amino acid residue sequence of ubiquinated murine legumain is shown in FIG. 19 (SEQ ID NO: 12). Protein expression of legumain was demonstrated by Western blotting with a polyclonal rabbit anti-murine legumain Ab as well as anti-murine beta-actin Ab (Santa Cruz Biotechnology, Inc) as a loading control. The specific protein was detected with a goat-anti-rabbit-HRP conjugated IgG Ab (Bio-Red Laboratories). Attenuated *Salmonella typhimurium* were transduced with DNA vaccine plasmids by electroporation as described in Luo et al. 2003, Proc. Natl. Acad. Sci. U.S.A 100:8850-8855 and Xiang et al. 2000, Proc. Natl. Acad. Sci. U.S.A 97:5492-5497.

### EXAMPLE 2.

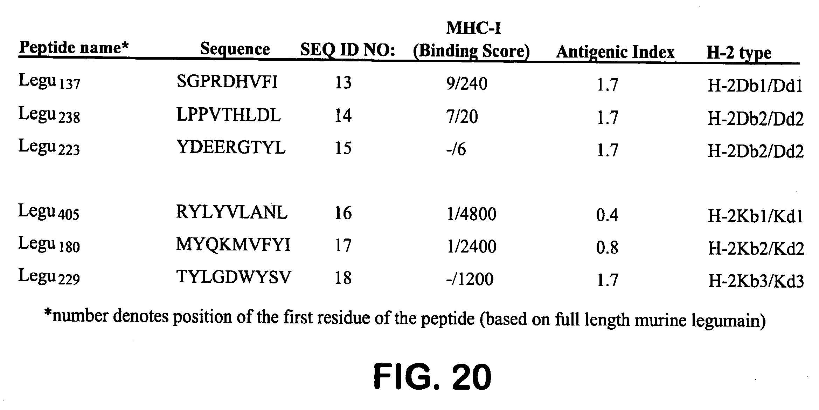

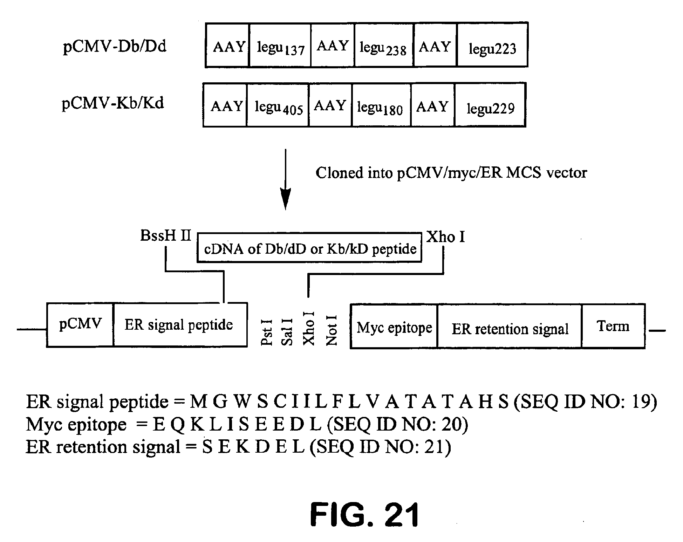

*Immunogenic murine legumain fragments.* Two plasmids, each comprising a legumain minigene encoding three immunogenic legumain fragments joined together by a three-amino acid spacer (AAY) between each fragment, were prepared by inserting the legumain minigene into a pCMV/myc/ER MCS plasmid, which is commercially available from Invitrogen, Carlsbad, CA (See FIG. 20 and 21). The vector includes a segment encoding an ER signal peptide, a myc epitope and an ER retention signal (see FIG. 21). The insertion of the minigene was made between a BssH II site in the ER signal peptide segment and a Xho I site, as shown in FIG. 21. The first legumain minigene plasmid (pCMV-Db/Dd; also referred to a pH-2Dd in the Figures) comprises an AAY spacer, immunogenic legumain fragment legu₁₃₇, an AAY spacer, immunogenic legumain fragment legu₂₃₈, an AAY spacer, and immunogenic legumain fragment legu₂₂₃. The second legumain minigene plasmid (pCMV-Kb/Kd; also referred to a pH-2Kd in the Figures) comprises an AAY spacer, immunogenic legumain fragment legu₄₀₅, an AAY spacer, immunogenic legumain fragment legu₁₈₀, an AAY spacer, and immunogenic legumain fragment legu₂₂₉. FIG. 20 shows the amino acid residue sequences for the immunogenic legumain fragments, as well as the MHC class I binding scores and sequence identifier number (SEQ ID NO) for each fragment. Peptide expression was verified by Western blotting of transfected COS-7 cells with monoclonal anti-myc antibody (Invitrogen). Once peptide expression was verified, a stop codon was introduced immediately in front of the *myc* epitope sequences of the vector. The resulting minigene plasmid vectors (pCMV-Db/Dd and pCMV-Kb/Kd) were each verified by nucleotide sequencing and then were transfected into attenuated *S. typhimurium* RE88 bacteria by electroporation to afford DNA compositions of the invention encoding legumain minigenes. As described in detail below, these compositions were effective for attacking 4T1 and D2F2 breast carcinoma in Balb/c mice. The observed tumor protective response was mediated by CD8 T cells, which specifically killed legumain⁺ tumor-associated macrophage cells, resulting in a marked suppression of tumor angiogenesis.

*Oral Immunization and Tumor Cell Challenge.* Groups of BALB/c mice (n=8) were treated 3 times at 1-week intervals by oral gavage with 100 µl PBS containing approximately 5x10⁸ CFU of doubly attenuated *S. typhimurium* harboring either empty vector, the pCMV-Db/Dd plasmid, or the pCMV-Kb/Kd plasmid. Mice were challenged i.v. or s.c. with D2F2 carcinoma cell lines 2 weeks after the last treatment.

*Cytotoxicity Assay.* Cytotoxicity was measured by a standard ⁵¹Cr-release assay as previously described. The percentage of specific target cell lysis was calculated by the formula [(E-S)/(T-S)] x 100, where E is the average experimental release, S the average spontaneous release, and T is the average total release.

*ELISPOT Assay.* Splenocytes were collected 2 weeks after D2F2 tumor cell challenge from all experimental groups of BALB/c mice, and cultured for 24 hours with either irradiated (1000Gy) 4T1 cells or 4T1 cells obtained from freshly harvested 4T1 breast tumor tissue. The assay was performed according to instructions provided by the manufacturer (BD Bioscience, San Jose, CA).

*Evaluation of anti-angiogenic activity.* Suppression of angiogenesis was determined by the Matrigel assay as previously described. Vessel growth in the Matrigel was determined by measuring the concentration of hemoglobin using Drabkin's reagent (aqueous solution containing 1 g/L NaHCO₃, 0.05 g/L KCN, and 0.2 g/L K₃Fe(CN)₆). Matrigel plugs were removed 6 days after Matrigel implantation, fixed in Bouin's solution (15 parts w/w saturated aqueous picric acid, 5 parts w/w of 37% aqueous formalin, and 1 part w/w of glacial acetic acid) for 24 hours, and embedded in paraffin. All tissues were sectioned, mounted onto slides and stained with Masson's trichrome. All images were captured by the SPOT™ Cooled Color digital camera system. Using the minigene approach of the present invention, the syngeneic BALB/c mice, anti-TAM minigene DNA compositions suppressed both tumor growth and angiogenesis. Six legumain immunogenic peptides were evaluated as H-2D^{d} -1,2,3, or H-2K^{d}- 1,2,3- restricted minigenes, based on the binding prediction of these MHC class I antigen molecules by the HLA Peptide Binding Predictions program provided by the BioInformatics & Molecular Analysis Section (BIMAS) of NIH website. The amino acid sequences of these peptides and their binding activities, as predicted by DNASTAR® software (DNAStar, Inc., Madison, WI), are listed in FIG. 20.

*The pCMV-Kb*/*Kd Minigene vaccine protects mice against D2F2 breast tumor cell challenge and prevents pulmonary metastasis.* Initially, the minigene DNA compositions were tested in prophylactic breast carcinoma model, where mice were first vaccinated with the minigene composition and then challenged s.c., with murine D2F2 breast carcinoma cells (FIG. 22). A marked inhibition of tumor growth was observed in syngeneic BALB/c mice vaccinated with pCMV-Kb/Kd, but not with pCMV-Db/Dd. In contrast, all mice vaccinated with only the empty vector control vector revealed rapid s.c. tumor growth.

The pCMV-Kb/Kd legumain minigene vaccine suppressed pulmonary D2F2 metastasis by attacking TAMs as indicated by the marked inhibition of experimental metastases observed in BALB/c mice challenged by i.v. injection of D2F2 breast carcinoma cells 2 weeks after the third vaccination. In contrast, pCMV-Db/Dd did prevent metastases in this model. Mice vaccinated with only the empty vector control revealed uniform, rapid metastatic pulmonary tumor growth (FIG 23).

*The minigene vaccine induces a CTL response which is capable of killing legumain*⁺ *cells.* In order to delineate the specific T cells response achieved after minigene vaccinations, T cell activation was demonstrated by the specific release of IFN-γ by activated T cells (FIG. 24, Panel B). These cells were stimulated with cells harvested from fresh 4T1 tumor tissue that highly expressed legumain as indicated by flow cytometry analysis (FIG. 24, Panel A). Importantly, cytotoxicity assays clearly demonstrated marked CTL activity only in immunized mice. The target cells were cells of murine macrophage cell line RAW, which had been cultured with IL-4, IL-10 and IL-13 cytokines to induce the expression of legumain. This was also verified by Western blot analysis (FIG. 25, Panel A), where wild type RAW cells were shown to be legumain-negative. Splenocytes isolated from control mice treated with empty vector showed similar background killing of RAW cells, either positive or negative for legumain expression (FIG. 25, Panel B). However, splenocytes from pCMV-Kb/Kd-treated mice induced significantly stronger killing against legumain⁺ target cells than against such cells harvested from mice treated with pCMV-Db/Dd or the empty vector (FIG. 25, Panel B). These data demonstrate the specificity of the pCMV-Kb/Kd minigene-induced CTL activity and its capability to specifically kill legumain⁺ macrophages.

The pCMV-Kb/Kd minigene vaccine induces anti-angiogenesis effects. In order to evaluate the extent to which anti-angiogenesis plays a key role in the pCMV-Kb/Kd-vaccine-induced tumor protection, Matrigel assays were performed in which blood vessel formation was induced within the Matrigel by recombinant bFGF. The difference in vessel formation between the various treatment groups was quantified by measuring the relative concentration of hemoglobin (Hb) in Matrigel plug extracts obtained from either immunized or control mice. Thus, mice treated with the pCMV-Kb/Kd vaccine displayed a clear reduction in the average relative concentration of Hb (FIG. 26, Panel A). Furthermore, when Matrigel sections from immunized and control mice were analyzed by Masson's trichrome staining, those obtained from mice in the empty vector control group revealed ample, multiple blood vessels. In contrast, blood vessels were markedly reduced in Matrigel sections from pCMV-Kb/Kd-treated mice (FIG. 26, Panel B). These data demonstrate that immunization with the pCMV-Kb/Kd vaccine resulted in the reduction of tumor vasculature. Taken together, these findings indicate that the pCMV-Kb/Kd minigene composition induced marked anti-angiogenic effects, which aided in the protection of BALB/c mice from challenges with D2F2 breast tumor cells in a prophylactic setting.

In separate experiments, with D121 non-small cell carcinoma cells in syngeneic C57/BL mice, both the pCMV-Kb/Kd and pCMV-Db/Dd compositions provided effective anti-TAM immune responses.

### Discussion.

*Legumain serves as a target to kill TAMs overexpressed during tumor progression.* It is well known that TAMs play an important role in tumor progression and metastasis. Therefore, targeting of these M2 macrophages represents a novel anti-tumor strategy. Legumain was initially identified as a significant marker molecule of TAMs, because of its high level of expression on these cells in the tumor microenvironment and stroma. TAMs were isolated from murine 4T1 breast tumor tissue. Flow cytometry (FACS) demonstrated that legumain was highly overexpressed on CD206 and F4/80 double positive M2 macrophages, especially when compared to normal M1 macrophages in the spleen (FIG. 1, Panel B). This result was also confirmed by immunohistochemical analyses indicating that TAMs could be visualized by H/E staining, and legumain overexpression was further indicated by double staining with anti-legumain antibody (Ab) (green) combined with anti-CD68 Ab (red) (FIG. 1, Panel A). These data demonstrate that infiltrating TAMs are a disproportionally large cell subpopulation in 4T1 tumor tissue and that legumain is a potentially effective target for killing TAMs.

*Induction of legumain expression on TAMs by Th2 cytokines.* A murine macrophage cell line, RAW, co-cultured with these cytokines, was used to assess the extent to which legumain expression on TAMs was induced by Th2 cytokines, such as IL-4, IL-10 and IL-13. A significant increase in CD206⁺, F4/80⁺ expression by these RAW cells was observed, concurrent with an upregulation of legumain (FIG. 1, Panel C). These results were confirmed by Western blotting (FIG. 1, Panel D). No evidence for legumain expression by tumor cell lines was found when cultured with these same cytokines. These findings indicate that Th2 cytokines, such as IL-4, IL-10 and IL-13, are released by tumor and other tumor stromal cells and accumulate in the tumor microenvironment. In this environment, the cytokines can potentially induce the proliferation and transformation from M1 macrophages to a population with M2 phenotype, which overexpresses legumain.

*Targeting of TAMs suppresses tumor progression.* Growth and metastases of tumors are highly coordinated with the presence ofTAMs, and therefore targeting of this macrophage subpopulation leads to suppression in tumor growth and metastases. An expression vector for a DNA composition encoding legumain was prepared to induce an immune response against these TAMs. Attenuated *Salmonella typhimurium* bacteria (strain RE88) were transfected with a plasmid vector encoding legumain to provide a delivery vehicle for legumain DNA to immune cells. A control composition was prepared by incorporating an empty plasmid vector into the same strain of attenuated bacteria. FIG. 2, Panel A schematically depicts a vector construction map based on the pCMV/myc/cyto vector backbone, incorporating DNA encoding legumain. The gene encoding legumain was fused to the C-terminus of mutant polyubiquitin (pLegumain) to improve antigen processing in the proteasome. The entire fragment was then inserted between the PstI and NotI restriction sites of the plasmid. Legumain expression was demonstrated by Western blotting. In both prophylactic and therapeutic setting, reducing the number of TAMs using a legumain-based DNA composition of the present invention effectively inhibited spontaneous 4T1 breast cancer metastases and metastases of D121 non-small cell lung and CT26 colon carcinomas in mouse models.

In a prophylactic setting, C57BL/6J mice were immunized three times with either phosphate buffered saline (PBS; control group 1), attenuated *S. typhimurium* incorporating the empty vector (control group 2) or a DNA composition of the invention (pLegumain-transfected attenuated *S. typhimurium;* treatment group). One week after the last immunization, these mice were challenged intravenously (i.v.) with about 2×10⁵ D121 non-small lung tumor cells. About 24 days thereafter, lung metastases were measured and analyzed. In the two control groups, the average lung weight was significantly greater than that of the treatment group (FIG. 2, Panel B). Similar results were obtained in the CT26 colon tumor model and the 4T1 breast cancer model in syngeneic BALB/c mice (FIG. 2, Panel B).

In a more demanding therapeutic setting, BALB/c mice were first challenged with 4T1 breast cancer cells and then immunized three times with the pLegumain-based DNA composition (treatment group), PBS (control), or an empty
vector composition (control), as described above. Twelve days after challenge with 4T1 tumor cells, the primary tumor was surgically excised. The life span curve for the control and treatment groups indicated that 75% (6/8) of the mice immunized with pLegumain survived for 3 months. In contrast, mice in the control groups all died within one month (FIG. 2, Panel C). These data indicate that the legumain-based DNA compositions of the present invention effectively suppress tumor cell growth and metastases in mouse models of 4T1 breast cancer, D121 non-small cell lung cancer and CT-26 colon carcinoma. Combined with surgery, this treatment could indeed significantly extend the life span of mice by inhibiting tumor cell metastases in these very challenging therapeutic mouse tumor models.

*Targeting legumain induces a specific CD8*+ *CTL response decreasing TAM populations in the tumor stroma.* Immunization against legumain induced a specific T cell response against TAMs that highly express this asparaginyl endopeptidase. The specific T cell response was demonstrated by a ⁵¹Cr release assay, where splenocytes isolated from mice successfully immunized with a DNA composition of the invention were effective in killing RAW macrophages, which expressed high levels of legumain after culture with cytokines IL-4, IL-10 and IL-13. These same splenocytes failed to induce cytotoxic killing of cells that did not express legumain (FIG. 3, Panel A), indicating a high degree of specificity for this T cell response against legumain. Additionally, the same result was obtained by using legumain transfected cells as targets in ⁵¹Cr release assays (FIG. 7). Furthermore, the results depicted in FIG. 3, Panel B demonstrate a dramatic decrease in the F4/80⁺/CD206⁺ macrophage population after treatment with a legumain-based DNA composition of the invention. These data were also confirmed by immunohistochemical staining, as shown in FIG. 3, Panel C.

*MHC-class I restricted CD8*⁺ *CTLs are specifically active against TAMs.* While not wishing to be bound by theory, it is believed that the transfected bacteria are taken up by Peyer's patches in the gut, where the legumain DNA is then incorporated into immune cells, such as macrophages and dendritic cells (DCs), which then express the legumain DNA. DCs in Peyer's patches of successfully immunized mice were found to be activated 3 days after vaccination with pLegumain, as indicated by the upregulated DC activation markers, CD40, CD80, and MHC-I (FIG. 4, Panel A). CD8⁺ T cell activation was also found to be specific for legumain, as indicated by double staining for INF-gamma and CD8 on splenocytes obtained from successfully vaccinated mice, (FIG. 4, Panel B), and by the specific release of INF- gamma by activated T cells stimulated with legumain-positive cells (FIG. 4, Panel C). In addition, *in vivo* immune depletion of CD4⁺ or CD8⁺ T cells revealed that only CD8⁺ T cells play a major role in the specific cytotoxic killing of TAMs since only their depletion completely abrogated this killing effect. This specific cytotoxity was MHC-class I antigen restricted, since killing was specifically inhibited by anti-H-2Dd/H-2Kd antibodies (FIG. 4, Panel D). Taken together, these results indicate that the legumain-based DNA compositions of the invention first activated DCs in Peyer's patches, after which these cells presented legumain peptides through the MHC-class I antigen pathway to the T cell receptor (TCR) on activated CD8⁺ T cells, resulting in a specific cytotoxic CD8⁺ T cell response abrogating TAMs.

*Abrogation of TAMs in the tumor stroma reduces the release of tumor growth factors and pro-angiogenesis factors, as well as inhibits tumor cell migration and metastases.* TAMs can influence tumor metastasis in several ways, since they secrete a wide variety of tumor growth factors, pro-angiogenesis factors, and tumor-associated enzymes that stimulate tumor angiogenesis, as well as tumor growth and metastasis. In an effort to assess the extent to which the elimination of TAMs actually reduced the release of some of these factors, serum and tumor tissue cells were collected from vaccinated mice and from suitable control animals. Freshly isolated tumor cells were cultured, and their supernatants collected at 24 and 48 hours, respectively. An ELISA, performed to quantify TNF-alpha, VEGF and TGF-beta, indicated a significant reduction in TNF-alpha and VEGF in both tumor cell supernatants and serum. TGF-beta was reduced only in cell supernatants, but not in serum (FIG. 5, Panel A).

Immunohistological staining confirmed a decrease in the expression of these factors in tumor tissue (FIG. 5, Panel B). In addition, a significant decrease in tumor cell migration was observed when comparing treatment and control groups (FIG. 5, Panel C) in a migration and invasion assay. This result indicates that the characteristics of tumor cells changed after the vaccine-induced remodeling of the tumor microenvironment caused by the reduction in TAMs. The ability to form tumor metastases was confirmed in an *in vivo* assay by determining a metastasis score and lung weights 24 days after primary tumor excision in a therapeutic setting. The metastasis score and lung weights for treated mice decreased significantly when compared with the two control groups (FIG. 5, Panel D).

*Elimination of TAMs in the tumor stroma results in reduction of tumor angiogenesis.* A marked anti-angiogenic effect was observed after eliminating TAMs in the tumor stroma, which may be in part due to the fact that these M2 macrophages produce a wide range of pro-angiogenesis factors. Matrigel assays detected new blood vessel grown *in vivo,* which was quantified by staining the endothelium with FITC-labeled isolectin B4. These results clearly show that vessel growth was significantly reduced after vaccination with a DNA composition of the present invention (FIG. 6, Panel B). A much greater number of blood vessels were observed to be growing in Matrigel plugs in control mice immunized with empty vector compared to those treated with the legumain DNA composition, as determined by digital imaging and staining with Masson Trichrome (FIG. 6, Panel A). Furthermore, an immunochemical histology assay was performed to assess the type of cells that actually migrated into the Matrigel plugs. Confocal microscopic images indicated that endothelial cells expressing CD31 or macrophages expressing CD68, grew or migrated to a considerable extent into Matrigel plugs in the empty vector control group, but did so to a considerable lesser extent in the treatment group (FIG. 6, Panel C).

*Transgenic legumain-expressing tumor cells are targeted by splenocytes from mice immunized with a DNA composition of the invention.* In a model study, the 4T1 cell line was stably transfected by a retrovirus harboring the legumain plasmid and then used as target cells (FIG. 7, Panel A, left photos have a 5x magnification; right photos have a 35x magnification). Photomicrograph images were taken 2 days after transfection. The positive cells are indicated by arrows inf FIG. 7, Panel A. A ⁵¹Cr release assay was performed; data are shown in FIG. 7, Panel B. Splenocytes isolated from mice immunized with the pLegumain DNA composition were effective in killing the 4T1 cells that were transfected with legumain (*P<0.01, compared to empty vector control groups). The tumor specific T cell-mediated killing was specific for legumain since 4T1 cells, lacking in legumain expression, were not lysed.

The present invention provides a new paradigm for tumor treatment, i.e., a reduction in the density of TAMs in the tumor stroma decreases the release of factors potentiating tumor growth and angiogenesis, which remodels the tumor micro-environment and markedly suppresses tumor cell proliferation, vascularization and metastasis. Targeting TAMs in the tumor stroma might raise the concern that abrogation of these cells could interfere with the normal immunological functions of these important components of the innate immune system. This is not the case, however.

Circulating monocytes are versatile precursors with the ability to differentiate into the various forms of specialized macrophages. In fact, the cytokine milieu profoundly affects the differentiation and function of tissue macrophages. Macrophages activated by bacterial products and Th1 cytokines are regarded as being of the M1 phenotype, i.e., classically activated macrophages with high bactericidal activity and cytotoxic function against tumor cells. In contrast, macrophages activated by Th2 cytokines, such as IL-4, and IL-13, or immunosuppressors, such as vitamin D3 and IL-10, are classified as having the M2 phenotype, which is characterized by a low cytotoxic function, but high tissue-remodeling activity.

M1 cells have immunostimulatory properties and defend the host against pathogenic infections, while M2 cells attenuate acute inflammatory reactions, potently scavenge cellular debris, and secrete a variety of pro-growth and angiogenesis factors essential for the repair of injured tissues. In addition, macrophages derived from healthy or inflamed tissue are capable of lysing tumor cells, expressing immunostimulatory cytokines, and presenting tumor-associated antigens to stimulate the proliferation and anti-tumor functions of T and NK cells. M2 macrophages such as TAMs, show reduced levels of these activities. This may be the result of their exposure to tumor-derived anti-inflammatory molecules such as IL-4, IL-10, TGF-beta1, and prostaglandin E2. Indeed, Mantovani and colleagues have suggested that exposure to IL-4 and IL-10 may induce monocytes in tumors to develop into polarized type II or M2 macrophages (Mantovani et al. 2002, Trends Immunol. 23:549-555).

To the extent that they have been previously investigated, differentiated mature TAMs have a phenotype and function similar to Type II macrophages (Mantovani et al. 2004, Eur. J. Cancer 40:1660-1667). Therefore, cytokines present in the tumor microenvironment have the potential to promote and orient the differentiation of recruited mononuclear phagocytes. Indeed, a growing body of evidence indicates that TAMs are skewed toward M2 macrophages in the tumor microenvironment, and produce a variety of pro-tumor growth and angiogenesis factors, as well as immunosuppressive molecules. Thus, the presence of TAMs at the tumor site and the continuous expression and release of their products may favor, rather than antagonize tumor progression and metastasis.

TAMs express abundant levels of CD206, a mannose receptor that is up-regulated on M2 macrophages following exposure to IL-4 and IL-13 (Porcheray et al. 2005, Clin. Exp. Immunol. 142:481-489). As demonstrated herein, this population of macrophages expresses a high level of legumain. Importantly, Th2 cytokines IL-4, IL-10 and IL-13 up-regulate the expression of CD206 and legumain on the macrophage cell line RAW. This finding can best be understood when one considers that macrophages are derived from peripheral blood ,and differentiate into M2 macrophages once they are recruited into tumor sites where IL-4, IL-13 and IL-10 are released by tumor cells and tumor stromal cells (Stein et al. 1992, J. Exp. Med. 176:287-292). Thus, targeting of M2 macrophages expressing legumain not only benefits suppression of tumor growth and metastases, but also maintains the normal functions of macrophages with M1 phenotype.

The relationship between infiltration by TAMs and prognosis in tumor patients has also been indicated by several studies, which concluded that the greater the macrophage infiltration, the worse the prognosis (see e.g., Wyckoff et al. 2004, Cancer Res. 64:7022-7029). Several lines of evidence indicate that a symbiotic relationship exists in the tumor stroma between cancer cells and TAMs, whereby cancer cells attract TAMs and sustain their survival, while TAMs respond to tumor-derived molecules by producing important growth factors and extracellular matrix enzymes. This, in turn, stimulates tumor proliferation, angiogenesis, and invasion of surrounding tissues (see e.g., Wyckoff et al. 2004, Cancer Res. 64:7022-7029). Thus, the attenuation of TAMs in the tumor environment provides an effective strategy to remodel the tumor stroma and to alter the tumor microenvironment.

DNA compositions of the present invention evoked a robust CTL response against this legumain, which is an asparaginyl endopeptidase that functions as a stress protein, and is highly overexpressed by TAMs. The and-legumain immune response was shown to be MHC-I class I antigen-restricted and CD8⁺ T cell specific. Importantly, the present invention demonstrates that after immunization with the legumain-based DNA composition of the invention, the density of double positive CD206⁺ and F4/80⁺ macrophages, i.e. TAMs, decreased dramatically. In addition, a variety of factors such as VEGF, MMP-9 and TGF-beta that are released by TAMs were shown to be present at low levels in both the supernatant of cultured tumor cells and mouse serum. It is well known that VEGF and metalloproteinase MMP-9 play important roles during the formation of the tumor vasculature and initiation of tumor angiogenesis. TAMs are significant in this regard, since they produce both VEGF and MMP-9. Progressively intensifying angiogenesis is associated with the upregulated expression of VEGF and extracellular proteases, such as MMP-9, whereas TGF-beta is known to be an important growth factor involved in the migration of tumor cells towards blood vessels. In fact, TGF- beta can provide proliferative and anti-apoptotic signals to tumor cells as well as activate urokinase-type plasminogen activators (uPA) that might contribute to the extracellular matrix breakdown which is required for vascular invasion to occur.

Significantly, the present invention demonstrates that once TAMs have been abrogated in the tumor tissue by specific CD8⁺ cytotoxic T lymphocytes (CTLs), the tumor cells changed their character by becoming less malignant and less invasive. The formation of a neovasculature in tumor tissues also was drastically reduced by treatment with a DNA composition of the present invention. Additionally, TAMs reportedly are involved in immune suppression and tolerance in the tumor microenvironment, and may inhibit T cell responses by inducing apoptosis of activated T cells via up-regulation of NO, PGs, TNF-alpha release and arginase activity (see e.g., Saio et al. 2001, J. Immunol. 167:5583-5593). After abrogation of TAMs, specific CD8⁺ T cell activity was markedly up-regulated, further indicating that the anti-TAM approach of the present invention provides an effective strategy to break immune tolerance against tumors.

In the 4T1 spontaneous mouse breast carcinoma metastasis model, a surprisingly significant increase in life span was obtained, in which 75% (6 out of 8) mice survived up to 3 months after 4T1 tumor cell inoculation into the mammary gland, once surgical resection of the primary tumor was followed by treatment with the legumain-based DNA composition of the invention. Even more unexpectedly, 62% (5 out of 8) mice revealed no lung metastases at all. Similar results were obtained in prophylactic settings in the other two tumor models, i.e. D121-non-small cell lung carcinoma and CT-26 colon carcinoma. These additional confirmatory data demonstrate that targeting of TAMs to remodel the tumor microenvironment is a potentially universal anti-tumor strategy, which suppresses tumor cell invasion and metastases by reducing the concentration of factors released by TAMs that otherwise promote tumor growth and metastasis.

In Example 2, a mammalian expression vector with an ER signal (pCMV/Myc/ER) was utilized for construction of a minigene DNA composition of the present invention. The ER signal peptide directs the protein into the secretory compartment. The vector also included a C-terminal peptide that retains the protein in the endoplasmic reticulum (ER). The peptides encoded by the DNA composition were processed in the ER of immune cells in the gut and then bound to MHC class I antigen binding sites to be fmally presented to T cell receptors, which induced a legumain-specific T cell response against TAMs in the mice which express legumain. These constructs create effective minigene vaccines which can induce an effective CD8⁺ T cell response against tumors of different organs in mouse tumor models.

Additional experiments with D121 non-small cell carcinoma cells in syngeneic C57/BL mice showed that both the pCMV-Db/Dd and pCMV-Kb/Kd minigene constructs provided effective anti-TAM immune response. In contrast, the results with D2F2 cells in BALB/c mice indicates that the pCMV-Kb/Kd construct was considerably more effective than either the pCMV-Db/Dd or the pCMV-Kb/Kd construct. A possible explanation for this finding is that there is a much greater number of legumain peptides predicted to bind to H-2Dd/Kd with higher affinity than those binding to H-2Dd/Kb molecules. According to the BioInformatics & Molecular Analysis Section (BIMAS) of NIH, the predictive scores of legumain peptides binding to H-2Dd and H-2Kd are considerably higher than the corresponding scores for H-2Db and H-2Kb, whereas the binding score of H-2Kd is the highest (FIG. 20).

Considering that the T cell receptor repertoire is almost unlimited, there may be a much higher number of peptide-H-2Dd/Kd complexes that can be recognized by the CTLs in the BALB/c mice. Moreover, the H-2D peptide had a much higher antigenicity index than two of three H-2K peptides (FIG. 20). Based on these data, antigenicity is less predictive than the MHC binding score with respect to the anti-TAM immune response. The legumain-based minigene compositions of the present invention induced an effective protection against tumors by attacking TAMs in breast tumor models. The tumor protection induced by the Kd-based minigene vaccines led to an attack on TAMs in the D2F2 breast carcinoma microenvironment in syngeneic in BALB/c mice. This protective immune response was found to be mediated by CD8⁺T cells, which specifically kill legumain⁺TAMs, and also results in a marked suppression of tumor angiogenesis. Importantly, the minigene DNA constructs of the invention proved to be of similar efficacy as a vaccine encoding the whole legumain gene. Similarly, both Dd and Kd-based constructs provided anti-TAM responses in a D121 cancer model in C57/BL mice. Taken together, these data represent the first anti-legumain minigene composition effective against TAMs and that this strategy can be particularly useful for individuals with different genetic background, and thereby provide a simple, more safe and flexible alternative to the whole-gene vaccine strategy in breast cancer treatment.

## Claims

1. A DNA composition comprising a DNA minigene construct that encodes for a polypeptide comprising a plurality of legumain fragments, said plurality consisting of three immunogenic fragments of legumain, each of which is preceded by the three amino acids AAY, the immunogenic fragments being joined together serially by said three amino acids AAY between each successive fragment in the polypeptide, wherein the polypeptide is capable of eliciting an immune response against tumor-associated cells, and is expressible in immune cells, and wherein the DNA minigene construct is incorporated in a pharmaceutically acceptable carrier, wherein said immunogenic fragments have an amino acid sequence according to SEQ ID NOs: 16, 17 and 18.

2. The DNA composition of claim 1, wherein the DNA minigene construct is a naked DNA construct.

3. The DNA composition of claim 2, wherein the naked DNA is in the form of a plasmid.

4. The DNA composition of any one of claims 1-3, wherein the DNA construct is incorporated in an attenuated viral vector or an attenuated bacterial vector.

5. The DNA composition of claim 4, wherein said attenuated vector is an attenuated AroA-, dam- Salmonella typhimurium.

6. The DNA composition of any one of claims 1-5, which further comprises a DNA construct encoding an immune effector protein expressible in immune cells.

7. The DNA composition of claim 6, wherein the immune effector protein is a cytokine.

8. The DNA composition of claim 7, wherein the immune effector protein is CCL21, IL-2 or CD40LT.

9. The DNA composition of any one of claims 1-8, wherein the DNA minigene construct further encodes an endoplasmic reticulum leader peptide sequence linked to the N-terminus of the polypeptide.

10. The DNA composition of any one of claims 1-9 for use in inhibiting tumor growth or tumor metastases in a mammal.

11. The DNA composition for use according to claim 10, wherein the mammal is a human.

12. The DNA composition for use according to any one of claims 10 and 11, wherein the composition is administered orally.

13. An article of manufacture comprising a DNA composition of any one of claims 1-9 packaged in a hermetically sealed, sterile container, the container having a label affixed thereto, the label bearing printed material identifying the composition and providing information useful to an individual administering said composition to a patient.

## Patentansprüche

1. DNA-Zusammensetzung umfassend ein DNA-Minigenkonstrukt, das für ein Polypeptid kodiert, das eine Pluralität von Legumainfragmenten umfasst, wobei die Pluralität aus drei immunogenen Legumainfragmenten besteht, denen jeweils die drei Aminosäuren AAY vorangestellt sind, wobei die immunogenen Fragmente seriell verbunden sind durch die drei Aminosäuren AAY zwischen jedem aufeinanderfolgenden Fragment im Polypeptid, wobei das Polypeptid imstande ist eine Immunantwort gegen Tumor-assoziierte Zellen auszulösen und in Immunzellen exprimierbar ist, und wobei das DNA-Minigenkonstrukt in einen pharmazeutisch verträglichen Träger eingebaut ist, wobei die immunogenen Fragmente eine Aminosäuresequenz gemäß SEQ ID NOs: 16, 17 und 18 aufweisen.

2. DNA-Zusammensetzung nach Anspruch 1, wobei das DNA-Minigenkonstrukt ein nacktes DNA-Konstrukt ist.

3. DNA-Zusammensetzung nach Anspruch 2, wobei die nackte DNA in Form eines Plasmids vorliegt.

4. DNA-Zusammensetzung nach einem der Ansprüche 1-3, wobei das DNA-Konstrukt in einen attenuierten viralen Vektor oder einen attenuierten bakteriellen Vektor eingebaut ist.

5. DNA-Zusammensetzung nach Anspruch 4, wobei der attenuierte Vektor ein attenuiertes AroA⁻, dam⁻ Salmonella typhimurium ist.

6. DNA-Zusammensetzung nach einem der Ansprüche 1-5, die weiterhin ein DNA-Konstrukt umfasst, das für ein in Immunzellen exprimierbares Immuneffektorprotein kodiert.

7. DNA-Zusammensetzung nach Anspruch 6, wobei das Immuneffektorprotein ein Cytokin ist.

8. DNA-Zusammensetzung nach Anspruch 7, wobei das Immuneffektorprotein CCL21, IL-2 oder CD40LT ist.

9. DNA-Zusammensetzung nach einem der Ansprüche 1-8, wobei das DNA-Minigenkonstrukt weiterhin eine Signalsequenz für das endoplasmatische Retikulum kodiert, die an den N-Terminus des Polypeptids gekoppelt ist.

10. DNA-Zusammensetzung nach einem der Ansprüche 1-9 zur Verwendung bei der Hemmung von Tumorwachstum oder Tumormetastasen in einem Säuger.

11. DNA-Zusammensetzung nach Anspruch 10, wobei der Säuger ein Mensch ist.

12. DNA-Zusammensetzung nach einem der Ansprüche 10 und 11, wobei die Zusammensetzung oral verabreicht wird.

13. Herstellungsprodukt umfassend die DNA-Zusammensetzung nach einem der Ansprüche 1-9, verpackt in einen hermetisch versiegelten, sterilen Behälter, wobei der Behälter mit einem daran befestigten Etikett versehen ist, wobei das Etikett bedrucktes Material trägt, das die Zusammensetzung identifiziert und Informationen liefert, die für eine Person nützlich sind, die die Zusammensetzung an einen Patienten verabreicht.

## Revendications

1. Composition d'ADN comprenant une construction de minigène d'ADN qui code pour un polypeptide comprenant une pluralité de fragments de légumaïne, ladite pluralité consistant en trois fragments immunogènes de légumaïne, dont chacun est précédé par les trois acides aminés AAY, les fragments immunogènes étant reliés entre eux en série par lesdits trois acides aminés AAY entre chacun des fragments successifs dans le polypeptide, tandis que le polypeptide est capable d'induire une réponse immune contre les cellules associées à une tumeur, et est apte à être exprimé dans des cellules immunes, et tandis que la construction de minigène d'ADN est incorporée dans un support pharmaceutiquement acceptable, lesdits fragments immunogènes ayant une séquence d'acides aminés selon SEQ ID NOs: 16, 17 et 18.

2. Composition d'ADN selon la revendication 1, dans laquelle la construction de minigène d'ADN est une construction d'ADN nu.

3. Composition d'ADN selon la revendication 2, dans laquelle l'ADN nu est sous la forme d'un plasmide.

4. Composition d'ADN selon l'une quelconque des revendications 1-3, dans laquelle la construction d'ADN est incorporée dans un vecteur viral atténué ou dans un vecteur bactérien atténué.

5. Composition d'ADN selon la revendication 4, dans laquelle ledit vecteur atténué est un thyphimurium d'AroA-,dam- Salmonelle atténué.

6. Composition d'ADN selon l'une quelconque des revendications 1-5, qui comprend en outre une construction d'ADN codant pour une protéine effectrice immune apte à être exprimée dans des cellules immunes.

7. Composition d'ADN selon la revendication 6, dans laquelle la protéine effectrice immune est une cytokine.

8. Composition d'ADN selon la revendication 7, dans laquelle la protéine effectrice immune est CCL21, IL-2 ou CD40LT.

9. Composition d'ADN selon l'une quelconque des revendications 1-8, dans laquelle la construction de minigène d'ADN code en outre pour une séquence peptidique de tête de réticulum endoplasmique liée à l'extrémité N-terminale du polypeptide.

10. Composition d'ADN selon l'une quelconque des revendications 1-9 pour utilisation dans l'inhibition de la croissance des tumeurs ou des métastases tumorales chez un mammifère.

11. Composition d'ADN pour utilisation selon la revendication 10, dans laquelle le mammifère est un humain.

12. Composition d'ADN pour utilisation selon l'une quelconque des revendications 10 et 11, dans laquelle la composition est administrée par voie orale.

13. Article manufacturé comprenant une composition d'ADN selon l'une quelconque des revendications 1-9 emballée dans un récipient stérile, hermétiquement fermé, le récipient ayant une étiquette qui y est fixée, l'étiquette portant un matériau imprimé identifiant la composition et procurant des informations utiles à un individu pour l'administration de ladite composition à un patient.


### SEQUENCE LISTING

<110> REISFELD, Ralph A. XIANG, Rong LUO, Yunping
<120> DNA COMPOSITION FOR ELICITING AN IMMUNE
   RESPONSE AGAINST TUMOR-ASSOCIATED MACROPHAGES
<130> TSRI 1215.1PCT
<150> US 60/849,927
   <151> 2006-10-06
<160> 21
<170> FastSEQ for Windows Version 4.0
<210> 1
   <211> 1302
   <212> DNA
   <213> Homo sapiens
<400> 1
   atggtttgga aagtagctgt attcctcagt gtggccctgg gcattggtgc cgttcctata 60
   gatgatcctg aagatggagg caagcactgg gtggtgatcg tggcaggttc aaatggctgg 120
   tataattata ggcaccaggc agacgcgtgc catgcctacc agatcattca ccgcaatggg 180
   attcctgacg aacagatcgt tgtgatgatg tacgatgaca ttgcttactc tgaagacaat 240
   cccactccag gaattgtgat caacaggccc aatggcacag gtgtctatca gggagtcccg 300
   aaggactaca ctggagagga tgttacccca caaaatttcc ttgctgtgtt gagaggcgat 360
   gcagaagcag tgaagggcat aggatccggc aaagtcctga agagtggccc ccaggatcac 420
   gtgttcattt acttcactga ccatggatct actggaatac tggtttttcc caatgaagat 480
   cttcatgtaa aggacctgaa tgagaccatc cattacatgt acaaacacaa aatgtaccga 540
   aagatggtgt tctacattga agcctgtgag tctgggtcca tgatgaacca cctgccggat 600
   aacatcaatg tttatgcaac tactgctgcc aaccccagag agtcgtccta cgcctgttac 660
   tatgatgaga agaggtccac gtacctgggg gactggtaca gcgtcaactg gatggaagac 720
   tcggacgtgg aagatctgac taaagagacc ctgcacaagc agtaccacct ggtaaaatcg 780
   cacaccaaca ccagccacgt catgcagtat ggaaacaaaa caatctccac catgaaagtg 840
   atgcagtttc agggtatgaa acgcaaagcc agttctcccg tccccctacc tccagtcaca 900
   caccttgacc tcacccccag ccctgatgtg cctctcacca tcatgaaaag gaaactgatg 960
   aacaccaatg atctggagga gtccaggcag ctcacggagg agatccagcg gcatctggat 1020
   gccaggcacc tcattgagaa gtcagtgcgt aagatcgtct ccttgctggc agcgtccgag 1080
   gctgaggtgg agcagctcct gtccgagaga gccccgctca cggggcacag ctgctaccca 1140
   gaggccctgc tgcacttccg gacccactgc ttcaactggc actcccccac gtacgagtat 1200
   gcgttgagac atttgtacgt gctggtcaac ctttgtgaga agccgtatcc acttcacagg 1260
   ataaaattgt ccatggacca cgtgtgcctt ggtcactatt aa                    1302
<210> 2
   <211> 433
   <212> PRT
   <213> Homo sapiens
<400> 2
<210> 3
   <211> 1308
   <212> DNA
   <213> Mus musculus
<400> 3
   atgacctgga gagtggctgt gcttctcagc ctggtgctgg gtgctggtgc cgttcccgtc 60
   ggtgtggacg atcccgagga tggaggcaag cactgggtgg tgattgtggc gggctccaat 120
   ggctggtata attaccgaca ccaggcagac gcatgccacg cctaccagat catccaccgg 180
   aacgggattc ctgacgagca gatcatagtg atgatgtatg acgacattgc caactctgaa 240
   gaaaacccta ccccaggtgt tgtgatcaac cgacctaacg gcacagatgt atacaaggga 300
   gtcctgaagg actacaccgg agaggatgtg actccagaga atttcctcgc cgtgctgaga 360
   ggtgacgcag aagctgtgaa gggcaaaggg tctggaaaag tcttgaagag tggcccccga 420
   gatcatgtct tcatttactt caccgaccac ggagccaccg ggatcctggt gtttcctaat 480
   gatgatcttc atgtcaagga cctgaataag actattcgct acatgtatga acacaaaatg 540
   taccagaaga tggtgttcta cattgaagct tgtgagtctg gctccatgat gaaccacctg 600
   cccgacgaca tcaacgttta tgcaactact gcggccaacc ccaaggagtc atcttatgcc 660
   tgctactacg acgaggagag gggcacttac ctgggtgact ggtacagcgt caactggatg 720
   gaagactccg atgtggagga cctgaccaaa gagacccttc acaagcagta ccacctggtc 780
   aagtcccaca ccaacaccag ccatgtcatg caatatggga acaaatctat ctctaccatg 840
   aaagtgatgc agtttcaggg aatgaagcac agagccagtt cccccatctc cctgcctccg 900
   gtcacacacc ttgacctcac ccccagccct gacgtgcccc tgaccatctt gaagaggaag 960
   ctgctgagaa ccaacgacgt gaaggaatcc cagaatctca ttgggcagat ccagcaattt 1020
   ctggatgcca ggcacgtcat tgagaagtct gtgcacaaga tcgtttccct gctggcggga 1080
   tttggggaaa ctgctgagag acatctgtca gagaggacca tgctcacagc acatgactgc 1140
   taccaggagg ctgtaaccca cttccgcaca cactgcttta actggcactc tgtcacgtac 1200
   gagcatgcct tgcggtactt gtatgtgctg gccaatctct gtgaggcacc atatccgatt 1260
   gacaggatag agatggccat ggacaaagtg tgtcttagtc actactga            1308
<210> 4
   <211> 435
   <212> PRT
   <213> Mus musculus
<400> 4
   Met Thr Trp Arg Val Ala Val Leu Leu Ser Leu Val Leu Gly Ala Gly
   1               5                   10                  15
   Ala Val Pro Val Gly Val Asp Asp Pro Glu Asp Gly Gly Lys His Trp
               20                  25                  30
   Val Val Ile Val Ala Gly Ser Asn Gly Trp Tyr Asn Tyr Arg His Gln
           35                  40                  45
   Ala Asp Ala Cys His Ala Tyr Gln Ile Ile His Arg Asn Gly Ile Pro
       50                  55                  60
   Asp Glu Gln Ile Ile Val Met Met Tyr Asp Asp Ile Ala Asn Ser Glu
   65                  70                  75                  80
   Glu Asn Pro Thr Pro Gly Val Val Ile Asn Arg Pro Asn Gly Thr Asp
                   85                  90                  95
   Val Tyr Lys Gly Val Leu Lys Asp Tyr Thr Gly Glu Asp Val Thr Pro
               100                 105                 110
   Glu Asn Phe Leu Ala Val Leu Arg Gly Asp Ala Glu Ala Val Lys Gly
           115                 120                 125
   Lys Gly Ser Gly Lys Val Leu Lys Ser Gly Pro Arg Asp His Val Phe
       130                 135                 140
   Ile Tyr Phe Thr Asp His Gly Ala Thr Gly Ile Leu Val Phe Pro Asn
   145                 150                 155                 160
   Asp Asp Leu His Val Lys Asp Leu Asn Lys Thr Ile Arg Tyr Met Tyr
                   165                 170                 175
   Glu His Lys Met Tyr Gln Lys Met Val Phe Tyr Ile Glu Ala Cys Glu
               180                 185                 190
   Ser Gly Ser Met Met Asn His Leu Pro Asp Asp Ile Asn Val Tyr Ala
           195                 200                 205
   Thr Thr Ala Ala Asn Pro Lys Glu Ser Ser Tyr Ala Cys Tyr Tyr Asp
       210                 215                 220
   Glu Glu Arg Gly Thr Tyr Leu Gly Asp Trp Tyr Ser Val Asn Trp Met
   225                 230                 235                 240
   Glu Asp Ser Asp Val Glu Asp Leu Thr Lys Glu Thr Leu His Lys Gln
                   245                 250                 255
   Tyr His Leu Val Lys Ser His Thr Asn Thr Ser His Val Met Gln Tyr
               260                 265                 270
   Gly Asn Lys Ser Ile Ser Thr Met Lys Val Met Gln Phe Gln Gly Met
           275                 280                 285
   Lys His Arg Ala Ser Ser Pro Ile Ser Leu Pro Pro Val Thr His Leu
       290                 295                 300
   Asp Leu Thr Pro Ser Pro Asp Val Pro Leu Thr Ile Leu Lys Arg Lys
   305                 310                 315                 320
   Leu Leu Arg Thr Asn Asp Val Lys Glu Ser Gln Asn Leu Ile Gly Gln
                   325                 330                 335
   Ile Gln Gln Phe Leu Asp Ala Arg His Val Ile Glu Lys Ser Val His
               340                 345                 350
   Lys Ile Val Ser Leu Leu Ala Gly Phe Gly Glu Thr Ala Glu Arg His
           355                 360                 365
   Leu Ser Glu Arg Thr Met Leu Thr Ala His Asp Cys Tyr Gln Glu Ala
       370                 375                 380
   Val Thr His Phe Arg Thr His Cys Phe Asn Trp His Ser Val Thr Tyr
   385                 390                 395                 400
   Glu His Ala Leu Arg Tyr Leu Tyr Val Leu Ala Asn Leu Cys Glu Ala
                   405                 410                 415
   Pro Tyr Pro Ile Asp Arg Ile Glu Met Ala Met Asp Lys Val Cys Leu
               420                 425                 430
   Ser His Tyr
           435
<210> 5
   <211> 814
   <212> DNA
   <213> Homo sapiens
<400> 5
   atcactctct ttaatcacta ctcacagtaa cctcaactcc tgccacaatg tacaggatgc 60
   aactcctgtc ttgcattgca ctaagtcttg cacttgtcac aaacagtgca cctacttcaa 120
   gttctacaaa gaaaacacag ctacaactgg agcatttact gctggattta cagatgattt 180
   tgaatggaat taataattac aagaatccca aactcaccag gatgctcaca tttaagtttt 240
   acatgcccaa gaaggccaca gaactgaaac atcttcagtg tctagaagaa gaactcaaac 300
   ctctggagga agtgctaaat ttagctcaaa gcaaaaactt tcacttaaga cccagggact 360
   taatcagcaa tatcaacgta atagttctgg aactaaaggg atctgaaaca acattcatgt 420
   gtgaatatgc tgatgagaca gcaaccattg tagaatttct gaacagatgg attacctttt 480
   gtcaaagcat catctcaaca ctgacttgat aattaagtgc ttcccactta aaacgtatca 540
   ggccttctat ttatttaaat atttaaattt tatatttatt gttgaatgta tggtttgcta 600
   cctattgtaa ctattattct taatcttaaa actataaata tggatctttt atgattcttt 660
   ttgtaagccc taggggctct aaaatggttt cacttattta tcccaaaata tttattatta 720
   tgttgaatgt taaatatagt atctatgtag attggttagt aaaactattt aataaatttg 780
   ataaatataa aaaaaaaaaa aaaaaaaaaa aaaa                             814
<210> 6
   <211> 153
   <212> PRT
   <213> Homo sapiens
<400> 6
   Met Tyr Arg Met Gln Leu Leu Ser Cys Ile Ala Leu Ser Leu Ala Leu
   1               5                   10                  15
   Val Thr Asn Ser Ala Pro Thr Ser Ser Ser Thr Lys Lys Thr Gln Leu
               20                  25                  30
   Gln Leu Glu His Leu Leu Leu Asp Leu Gln Met Ile Leu Asn Gly Ile
           35                  40                  45
   Asn Asn Tyr Lys Asn Pro Lys Leu Thr Arg Met Leu Thr Phe Lys Phe
               50                  55                  60
   Tyr Met Pro Lys Lys Ala Thr Glu Leu Lys His Leu Gln Cys Leu Glu
   65                  70                  75                  80
   Glu Glu Leu Lys Pro Leu Glu Glu Val Leu Asn Leu Ala Gln Ser Lys
                   85                  90                  95
   Asn Phe His Leu Arg Pro Arg Asp Leu Ile Ser Asn Ile Asn Val Ile
               100                 105                 110
   Val Leu Glu Leu Lys Gly Ser Glu Thr Thr Phe Met Cys Glu Tyr Ala
           115                 120                 125
   Asp Glu Thr Ala Thr Ile Val Glu Phe Leu Asn Arg Trp Ile Thr Phe
       130                 135                 140
   Cys Gln Ser Ile Ile Ser Thr Leu Thr
   145                 150
<210> 7
   <211> 852
   <212> DNA
   <213> Homo sapiens
<400> 7
   cttgcagctg cccacctcac cctcagctct ggcctcttac tcaccctcta ccacagacat 60
   ggctcagtca ctggctctga gcctccttat cctggttctg gcctttggca tccccaggac 120
   ccaaggcagt gatggagggg ctcaggactg ttgcctcaag tacagccaaa ggaagattcc 180
   cgccaaggtt gtccgcagct accggaagca ggaaccaagc ttaggctgct ccatcccagc 240
   tatcctgttc ttgccccgca agcgctctca ggcagagcta tgtgcagacc caaaggagct 300
   ctgggtgcag cagctgatgc agcatctgga caagacacca tccccacaga aaccagccca 360
   gggctgcagg aaggacaggg gggcctccaa gactggcaag aaaggaaagg gctccaaagg 420
   ctgcaagagg actgagcggt cacagacccc taaagggcca tagcccagtg agcagcctgg 480
   agccctggag accccaccag cctcaccaac gcttgaagcc tgaacccaag atgcaagaag 540
   gaggctatgc tcaggggccc tggagcagcc accccatgct ggccttgcca cactctttct 600
   cctgctttaa ccaccccatc tgcattccca gctctaccct gcatggctga gctgcccaca 660
   gcaggccagg tccagagaga ccgaggaggg agagtctccc agggagcatg agaggaggca 720
   gcaggactgt ccccttgaag gagaatcatc aggaccctgg acctgatacg gctccccagt 780
   acaccccacc tcttccttgt aaatatgatt tatacctaac tgaataaaaa gctgttctgt 840
   cttcccaccc gc                                                     852
<210> 8
   <211> 134
   <212> PRT
   <213> Homo sapiens
<400> 8
   Met Ala Gln Ser Leu Ala Leu Ser Leu Leu Ile Leu Val Leu Ala Phe
   1               5                   10                  15
   Gly Ile Pro Arg Thr Gln Gly Ser Asp Gly Gly Ala Gln Asp Cys Cys
               20                  25                  30
   Leu Lys Tyr Ser Gln Arg Lys Ile Pro Ala Lys Val Val Arg Ser Tyr
           35                  40                  45
   Arg Lys Gln Glu Pro Ser Leu Gly Cys Ser Ile Pro Ala Ile Leu Phe
       50                  55                  60
   Leu Pro Arg Lys Arg Ser Gln Ala Glu Leu Cys Ala Asp Pro Lys Glu
   65                  70                  75                  80
   Leu Trp Val Gln Gln Leu Met Gln His Leu Asp Lys Thr Pro Ser Pro
                   85                  90                  95
   Gln Lys Pro Ala Gln Gly Cys Arg Lys Asp Arg Gly Ala Ser Lys Thr
               100                 105                 110
   Gly Lys Lys Gly Lys Gly Ser Lys Gly Cys Lys Arg Thr Glu Arg Ser
           115                 120                 125
   Gln Thr Pro Lys Gly Pro
   130
<210> 9
   <211> 1816
   <212> DNA
   <213> Homo sapiens
<400> 9
   cttctctgcc agaagatacc atttcaactt taacacagca tgatcgaaac atacaaccaa 60
   acttctcccc gatctgcggc cactggactg cccatcagca tgaaaatttt tatgtattta 120
   cttactgttt ttcttatcac ccagatgatt gggtcagcac tttttgctgt gtatcttcat 180
   agaaggttgg acaagataga agatgaaagg aatcttcatg aagattttgt attcatgaaa 240
   acgatacaga gatgcaacac aggagaaaga tccttatcct tactgaactg tgaggagatt 300
   aaaagccagt ttgaaggctt tgtgaaggat ataatgttaa acaaagagga gacgaagaaa 360
   gaaaacagct ttgaaatgca aaaaggtgat cagaatcctc aaattgcggc acatgtcata 420
   agtgaggcca gcagtaaaac aacatctgtg ttacagtggg ctgaaaaagg atactacacc 480
   atgagcaaca acttggtaac cctggaaaat gggaaacagc tgaccgttaa aagacaagga 540
   ctctattata tctatgccca agtcaccttc tgttccaatc gggaagcttc gagtcaagct 600
   ccatttatag ccagcctctg cctaaagtcc cccggtagat tcgagagaat cttactcaga 660
   gctgcaaata cccacagttc cgccaaacct tgcgggcaac aatccattca cttgggagga 720
   gtatttgaat tgcaaccagg tgcttcggtg tttgtcaatg tgactgatcc aagccaagtg 780
   agccatggca ctggcttcac gtcctttggc ttactcaaac tctgaacagt gtcaccttgc 840
   aggctgtggt ggagctgacg ctgggagtct tcataataca gcacagcggt taagcccacc 900
   ccctgttaac tgcctattta taaccctagg atcctcctta tggagaacta tttattatac 960
   actccaaggc atgtagaact gtaataagtg aattacaggt cacatgaaac caaaacgggc 1020
   cctgctccat aagagcttat atatctgaag cagcaacccc actgatgcag acatccagag 1080
   agtcctatga aaagacaagg ccattatgca caggttgaat tctgagtaaa cagcagataa 1140
   cttgccaagt tcagttttgt ttctttgcgt gcagtgtctt tccatggata atgcatttga 1200
   tttatcagtg aagatgcaga agggaaatgg ggagcctcag ctcacattca gttatggttg 1260
   actctgggtt cctatggcct tgttggaggg ggccaggctc tagaacgtct aacacagtgg 1320
   agaaccgaaa cccccccccc cccccccgcc accctctcgg acagttattc attctctttc 1380
   aatctctctc tctccatctc tctctttcag tctctctctc tcaacctctt tcttccaatc 1440
   tctctttctc aatctctctg tttccctttg tcagtctctt ccctccccca gtctctcttc 1500
   tcaatccccc tttctaacac acacacacac acacacacac acacacacac acacacacac 1560
   acacacacac acacacacac agagtcaggc cgttgctagt cagttctctt ctttccaccc 1620
   tgtccctatc tctaccacta tagatgaggg tgaggagtag ggagtgcagc cctgagcctg 1680
   cccactcctc attacgaaat gactgtattt aaaggaaatc tattgtatct acctgcagtc 1740
   tccattgttt ccagagtgaa cttgtaatta tcttgttatt tattttttga ataataaaga 1800
   cctcttaaca ttaaaa                                                  1816
<210> 10
   <211> 261
   <212> PRT
   <213> Homo sapiens
<400> 10
<210> 11
   <211> 1533
   <212> DNA
   <213> Artificial Sequence
<220>
   <223> Synthetic ubiquinated murine legumain
<400> 11
   atgcagatct tcgtgaagac cctgaccggc aagaccatca ccctagaggt ggagcccagt 60
   gacaccatcg agaacgtgaa ggccaagatc caggataaag agggcatccc ccctgaccag 120
   cagaggctga tctttgccgg caagcagctg gaagatggcc gcaccctctc tgattacaac 180
   atccagaagg agtcaaccct gcacctggtc cttcgcctga gaggtggcac ctggagagtg 240
   gctgtgcttc tcagcctggt gctgggtgct ggtgccgttc ccgtcggtgt ggacgatccc 300
   gaggatggag gcaagcactg ggtggtgatt gtggcgggct ccaatggctg gtataattac 360
   cgacaccagg cagacgcatg ccacgcctac cagatcatcc accggaacgg gattcctgac 420
   gagcagatca tagtgatgat gtatgacgac attgccaact ctgaagaaaa ccctacccca 480
   ggtgttgtga tcaaccgacc taacggcaca gatgtataca agggagtcct gaaggactac 540
   accggagagg atgtgactcc agagaatttc ctcgccgtgc tgagaggtga cgcagaagct 600
   gtgaagggca aggggtctgg aaaagtcttg aagagtggcc cccgagatca tgtcttcatt 660
   tacttcaccg accacggagc caccgggatc ctggtgtttc ctaatgatga tcttcatgtc 720
   aaggacctga ataagactat tcgctacatg tatgaacaca aaatgtacca gaagatggtg 780
   ttctacattg aagcttgtga gtctggctcc atgatgaacc acctgcccga cgacatcaac 840
   gtttatgcaa ctactgcggc caaccccaag gagtcatctt atgcctgcta ctacgacgag 900
   gagaggggca cttacctggg tgactggtac agcgtcaact ggatggaaga ctccgatgtg 960
   gaggacctga ccaaagagac ccttcacaag cagtaccacc tggtcaagtc ccacaccaac 1020
   accagccatg tcatgcaata tgggaacaaa tctatctcta ccatgaaagt gatgcagttt 1080
   cagggaatga agcacagagc cagttccccc atctccctgc ctccggtcac acaccttgac 1140
   ctcaccccca gccctgacgt gcccctgacc atcttgaaga ggaagctgct gagaaccaac 1200
   gacgtgaagg aatcccagaa tctcattggg cagatccagc aatttctgga tgccaggcac 1260
   gtcattgaga agtctgtgca caagatcgtt tccctgctgg cgggatttgg ggaaactgct 1320
   gagagacatc tgtcagagag gaccatgctc acagcacatg actgctacca ggaggctgta 1380
   acccacttcc gcacacactg ctttaactgg cactctgtca cgtacgagca tgccttgcgg 1440
   tacttgtatg tgctggccaa tctctgtgag gcaccatatc cgattgacag gatagagatg 1500
   gccatggaca aagtgtgtct tagtcactac tga                              1533
<210> 12
   <211> 510
   <212> PRT
   <213> Artificial Sequence
<220>
   <223> Synthetic ubiquinated murine legumain
<400> 12
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Arg Leu Ile Phe Ala Gly Lys
   35 40 45
   Gln Leu Glu Asp Gly Arg Thr Leu Ser Asp Tyr Asn Ile Gln Lys Glu
   50 55 60
   Ser Thr Leu His Leu Val Leu Arg Leu Arg Gly Gly Thr Trp Arg Val
   65 70 75 80
   Ala Val Leu Leu Ser Leu Val Leu Gly Ala Gly Ala Val Pro Val Gly
   85 90 95
   Val Asp Asp Pro Glu Asp Gly Gly Lys His Trp Val Val Ile Val Ala
   100 105 110
   Gly Ser Asn Gly Trp Tyr Asn Tyr Arg His Gln Ala Asp Ala Cys His
   115 120 125
   Ala Tyr Gln Ile Ile His Arg Asn Gly Ile Pro Asp Glu Gln Ile Ile
   130 135 140
   Val Met Met Tyr Asp Asp Ile Ala Asn Ser Glu Glu Asn Pro Thr Pro
   145 150 155 160
   Gly Val Val Ile Asn Arg Pro Asn Gly Thr Asp Val Tyr Lys Gly Val
   165 170 175
   Leu Lys Asp Tyr Thr Gly Glu Asp Val Thr Pro Glu Asn Phe Leu Ala
   180 185 190
   Val Leu Arg Gly Asp Ala Glu Ala Val Lys Gly Lys Gly Ser Gly Lys
   195 200 205
   Val Leu Lys Ser Gly Pro Arg Asp His Val Phe Ile Tyr Phe Thr Asp
   210 215 220
   His Gly Ala Thr Gly Ile Leu Val Phe Pro Asn Asp Asp Leu His Val
   225 230 235 240
   Lys Asp Leu Asn Lys Thr Ile Arg Tyr Met Tyr Glu His Lys Met Tyr
   245 250 255
   Gln Lys Met Val Phe Tyr Ile Glu Ala Cys Glu Ser Gly Ser Met Met
   260 265 270
   Asn His Leu Pro Asp Asp Ile Asn Val Tyr Ala Thr Thr Ala Ala Asn
   275 280 285
   Pro Lys Glu Ser Ser Tyr Ala Cys Tyr Tyr Asp Glu Glu Arg Gly Thr
   290 295 300
   Tyr Leu Gly Asp Trp Tyr Ser Val Asn Trp Met Glu Asp Ser Asp Val
   305 310 315 320
   Glu Asp Leu Thr Lys Glu Thr Leu His Lys Gln Tyr His Leu Val Lys
   325 330 335
   Ser His Thr Asn Thr Ser His Val Met Gln Tyr Gly Asn Lys Ser Ile
   340 345 350
   Ser Thr Met Lys Val Met Gln Phe Gln Gly Met Lys His Arg Ala Ser
   355 360 365
   Ser Pro Ile Ser Leu Pro Pro Val Thr His Leu Asp Leu Thr Pro Ser
   370 375 380
   Pro Asp Val Pro Leu Thr Ile Leu Lys Arg Lys Leu Leu Arg Thr Asn
   385 390 395 400
   Asp Val Lys Glu Ser Gln Asn Leu Ile Gly Gln Ile Gln Gln Phe Leu
   405 410 415
   Asp Ala Arg His Val Ile Glu Lys Ser Val His Lys Ile Val Ser Leu
                   420                 425                 430
   Leu Ala Gly Phe Gly Glu Thr Ala Glu Arg His Leu Ser Glu Arg Thr
           435                 440                 445
   Met Leu Thr Ala His Asp Cys Tyr Gln Glu Ala Val Thr His Phe Arg
       450                 455                 460
   Thr His Cys Phe Asn Trp His Ser Val Thr Tyr Glu His Ala Leu Arg
   465                 470                 475                 480
   Tyr Leu Tyr Val Leu Ala Asn Leu Cys Glu Ala Pro Tyr Pro Ile Asp
                   485                 490                 495
   Arg Ile Glu Met Ala Met Asp Lys Val Cys Leu Ser His Tyr
                   500                 505                 510
<210> 13
   <211> 9
   <212> PRT
   <213> Mus musculus
<400> 13
   Ser Gly Pro Arg Asp His Val Phe Ile
    1               5
<210> 14
   <211> 9
   <212> PRT
   <213> Mus musculus
<400> 14
   Leu Pro Pro Val Thr His Leu Asp Leu
    1               5
<210> 15
   <211> 9
   <212> PRT
   <213> Mus musculus
<400> 15
   Tyr Asp Glu Glu Arg Gly Thr Tyr Leu
    1               5
<210> 16
   <211> 9
   <212> PRT
   <213> Mus musculus
<400> 16
   Arg Tyr Leu Tyr Val Leu Ala Asn Leu
    1               5
<210> 17
   <211> 9
   <212> PRT
   <213> Mus musculus
<400> 17
   Met Tyr Gln Lys Met Val Phe Tyr Ile
   1 5
<210> 18
   <211> 9
   <212> PRT
   <213> Mus musculus
<400> 18
   Thr Tyr Leu Gly Asp Trp Tyr Ser Val
   1 5
<210> 19
   <211> 18
   <212> PRT
   <213> Mus musculus
<400> 19
   Met Gly Trp Ser Cys Ile Ile Leu Phe Leu Val Ala Thr Ala Thr Ala
   1 5 10 15
   His Ser
<210> 20
   <211> 10
   <212> PRT
   <213> Mus musculus
<400> 20
   Glu Gln Lys Leu Ile Ser Glu Glu Asp Leu
   1 5 10
<210> 21
   <211> 6
   <212> PRT
   <213> Mus musculus
<400> 21
   Ser Glu Lys Asp Glu Leu
   1 5